# EUROPEAN PATENT APPLICATION

(11) **EP 4 046 658 A1**
(43) Date of publication of application: **24.08.2022**
(21) Application number: 20863556.5
(22) Date of filing: 10.09.2020
(51) Int. Cl.: A61K 45/00, A61K 38/30, A61K 38/18, A61P 37/02, A61P 29/00

(54) **APPLICATION OF NON-IGF1R-BINDING SUBSTANCE IN PREVENTION AND/OR TREATMENT OF INFLAMMATORY DISEASES**

(30) Priority: 10.09.2019 CN 201910854478
(71) Applicant: Shanghai Institute of Nutrition and Health, Chinese Academy of Sciences, Shanghai 200031 (CN)
(72) Inventor: SHI, Yufang, Shanghai 200031 (CN); WANG, Ying, Shanghai 200031 (CN); WANG, Xuefeng, Shanghai 200031 (CN)
(74) Representative: Rondano, Davide
(86) International application number: PCT/CN2020/114587
(87) International publication number: WO 2021/047607

(57) **Abstract**

Provided is an application of a non-IGF1R-binding substance in the prevention and/or treatment of inflammatory diseases. Specifically, provided is use of a non-IGF1R-binding substance. The non-IGF1R-binding substance is used for preparing a composition or formulation, and the composition or formulation is used for preventing and/or treating inflammatory diseases.

## Description

### Technical field

The present invention relates to the field of biomedicine, in particular, to the application of non-IGF1R-binding substances in preventing and/or treating inflammatory diseases.

### Background

Insulin-like growth factor 2 (IGF2) belongs to the insulin-like growth factor family, involved with other families in the regulation of cell proliferation, differentiation, metabolism, aging and death, and affects the development, growth, health and disease of organisms^{1, 2}. IGF2 receptors include IGF1R and IGF2R, which have the highest affinity with IGF2R, followed by binding to IGF1R, and hardly bind to insulin receptors².

Current studies suggest that the main biological function of IGF2 is turned on by tyrosine kinases that activate IGF1R, and there is no positive correlation with IGF2R and IR².

About 90% of IGF2R is distributed in the intracellular dictyosome, and the remaining 10% of IGF2R is distributed in the the outer membrane of a cell³. IGF2R is not the only receptor of IGF2, nor is IGF2 the only ligand of IGF2R. Its ligands also include mannose-6-phosphate. In existing reports, the main function of IGF2R is to transport its ligands to lysosomes to complete protein processing or degradation⁴. In addition, studies have found that IGF2R on the cell membrane can be cleaved in a soluble form into the blood and other fluid environments to effectively bind to IGF2 and inhibit the function of IGF2^{4,5}.

Autoimmune diseases are diseases in which the immune system abnormally attacks its own organs and tissue cells. According to the data released by the US Department of Health and Human Services, there are currently 24 million Americans - 7% of the US population suffers from more than eighty autoimmune diseases, involving almost every part of the body ^{6,7}. The pathogenic factor of most autoimmune diseases is still unclear, may be related to genetic, infection, drug and environmental factors. The course of the disease is longer, with repeated remissions and attacks, and more women than men are affected. Most autoimmune diseases involve the involvement of phagocytes composed of mononuclear precursor cells, monocytes and macrophages.

In various autoimmune diseases, activated macrophages secrete Milk fat globule-epidermal growth factor 8 (MFG-E8), the C-terminal domain of MFG-E8 a binds phosphatidylserine in apoptotic cells, mediates the phagocytosis of apoptotic cells by macrophages, clears apoptotic cells that have been attacked and dead immune cell debris in autoimmune diseases, and helps the body to restore homeostasis⁸. Some inflammatory factors, such as lipopolysaccharide, can inhibit the expression of MFG-E8 in macrophages, inhibit the clearance of apoptotic debris and aggravate the inflammatory response⁹. On the contrary, MFG-E8-mediated macrophage phagocytosis of apoptotic debris can also inhibit the regulation of pro-inflammatory effects of lipopolysaccharides on macrophages, including the inhibition of p38, ERK1/2, c-JNK, MAPK and p65¹⁰.

Inflammation is the body's defense response to its own and foreign pro-inflammatory factors. Pro-inflammatory factors include biological factors, physical factors, chemical factors, foreign bodies, necrotic tissue, allergic reactions and pathological changes. The inflammatory response is regulated by pro-inflammatory factors and the body itself ¹¹. In inflammatory diseases, both innate immunity and adaptive immunity are involved. In the early stage of inflammation, innate immunity first reaches the site of tissue damage, affecting and determining the response types of innate and adaptive immunity in the later stage¹². When there is a disorder in the regulation of inflammation, an inflammatory disease occurs, the ability to resist stimuli and pathogens is lost, and the disease progression is accelerated. According to the speed of the inflammatory response, the inflammatory response includes acute and chronic inflammatory responses.

At present, there are two main classes of drugs for the treatment of inflammatory diseases and autoimmune diseases: the first class is steroidal anti-inflammatory drugs, that is, steroid drugs, such as adrenocortical hormone, androgens, and estrogens, having certain anti-inflammatory effects, long-term use will cause multi-system dysfunction such as endocrine disorders¹³⁻¹⁵. The second class is NSAIDs including aspirin, acetaminophen, indomethacin, naproxen, nabumetone, diclofenac, ibuprofen, nimesulide, rofecoxib, celecoxib etc., it is widely used in clinical anti-inflammatory treatment such as osteoarthritis and rheumatoid arthritis¹⁴⁻¹⁶. NSAIDs have a single mechanism of action, mainly by inhibiting the synthesis of prostaglandins, reducing the formation of bradykinin, and reducing the aggregation and agglutination of leukocytes and platelets to exert anti-inflammatory effects¹⁷. Therefore, steroid drugs are limited by side effects, while non-steroid drugs are limited by a single pathway of action, and the sensitization effect of combined drugs is not good.

Therefore, there is an urgent need in the art to develop a drug that can more effectively treat autoimmune diseases and/or inflammatory diseases.

### Summary of the invention

The purpose of the present invention is to provide a medicine that can more effectively treat autoimmune diseases and/or inflammatory diseases.

In a first aspect of the present invention, it provides a use of a non-IGF1R-binding substance for the preparation of a composition or preparation for preventing and/or treating inflammatory diseases.

In another preferred embodiment, the non-IGF1R-binding substance is selected from the group consisting of an IGF2 mutant, a vector expressing an IGF2 mutant, an antibody, a small molecule compound, and a combination thereof.

In another preferred embodiment, the inflammatory disease is selected from the group consisting of: peritonitis, inflammatory bowel disease, multiple sclerosis, diabetes, systemic lupus erythematosus, scleroderma, hashimoto thyroiditis, autoimmune hepatitis, autoimmune uveitis, interstitial lung disease, psoriasis, leukoderma, dermatomyositis, kawasaki disease, adult ear disease, ankylosing spondylitis, sarcoidosis, enthesitis-related arthritis, Polyarticular Course Juvenile Idiopathic Arthritis, rheumatoid arthritis, graft-versus-host disease, autoimmune pancreatitis, Parkinson's disease, Alzheimer's disease, and a combination thereof.

In another preferred embodiment, the non-IGF1R-binding substances include substances with higher selectivity or affinity for IGF2R than IGF1R, and substances that do not activate or substantially do not activate IGF1R that can efficiently activate IGF2R (such as non-IGFIR ligands such as retinoic acid).

In another preferred embodiment, the vector for expressing the IGF2 mutant includes a viral vector.

In another preferred embodiment, the viral vector is selected from the group consisting of adenoviral vector, lentiviral vector, and a combination thereof.

In another preferred embodiment, the non-IGF1R-binding substance further includes one or more substances selected from the group consisting of: profibrinolysin (Plasminogen), Serglycin, Cellular repressor of E1A-stimulated genes (CREG), sulfamidase (Sgsh), Phosphorylated β-glucuronidase (Gusb) and other effective ligands of IGF2R.

In another preferred embodiment, the IGF2 mutant is mutated at the tyrosine corresponding to position 27 of SEQ ID NO.: 1 of the wild-type IGF2 protein.

In another preferred embodiment, the tyrosine at position 27 is mutated to one or more amino acids selected from the group consisting of leucine, isoleucine, valine, methionine, alanine, phenylalanine, serine, proline, threonine, histidine, lysine, tryptophan, arginine, glutamic acid, glycine, aspartic acid, cysteine.

In another preferred embodiment, the tyrosine at position 27 is mutated to leucine.

In another preferred embodiment, the IGF2 mutant is mutated to leucine at the tyrosine corresponding to position 27 of SEQ ID NO.: 1 of the wild-type IGF2 protein and optional deletion of the D-domain (threonine on position 62 to glutamic acid on position 67 of SEQ ID NO.: 1).

In another preferred embodiment, the IGF2 mutant is mutated at the glutamic acid corresponding to the position 12 of SEQ ID NO.: 1 of the wild-type IGF2 protein, and optional deletion of the D-domain (threonine on position 62 to glutamic acid on position 67 of SEQ ID NO.: 1).

In another preferred embodiment, the glutamic acid at position 12 is mutated to one or more amino acids selected from the group consisting of:
Asp, Ala, Gln, His, Arg, Lys.

In another preferred embodiment, the IGF2 mutant is mutated at the phenylalanine corresponding to position 26 of SEQ ID NO.: 1 of the wild-type IGF2 protein, and optional deletion of the D-domain (threonine on position 62 to glutamic acid on position 67 of SEQ ID NO.: 1).

In another preferred embodiment, the phenylalanine at position 26 is mutated to one or more amino acids selected from the group consisting of Ser, Asp, Ala, Gln, His, Arg, and Lys.

In another preferred embodiment, the phenylalanine at position 26 is mutated to serine.

In another preferred embodiment, the IGF2 mutant is mutated at the tyrosine corresponding to the position 27 of SEQ ID NO.: 1 of the wild-type IGF2 protein, and the D-domain is optionally deleted (threonine on position 62 to glutamic acid on position 67 of SEQ ID NO.: 1).

In another preferred embodiment, the tyrosine at position 27 is mutated to one or more amino acids selected from the group consisting of Leu, Asp, Ala, Gln, His, Arg, and Lys.

In another preferred embodiment, the tyrosine at position 27 is mutated to leucine.

In another preferred embodiment, the IGF2 mutant is mutated at the valine corresponding to the position 43 of SEQ ID NO.: 1 of the wild-type IGF2 protein, and the D-domain is optionally deleted (threonine on position 62 to glutamic acid on position 67 of SEQ ID NO.: 1).

In another preferred embodiment, the valine at position 43 is mutated to one or more amino acids selected from the group consisting of Leu, Asp, Ala, Gln, His, Arg, and Lys.

In another preferred embodiment, the valine at position 43 is mutated to leucine.

In another preferred embodiment, the tyrosine at position 27 is mutated to one or more amino acids selected from the group consisting of Leu, Asp, Ala, Gln, His, Arg, Lys, and at the same time the valine at position 43 is mutated to one or more amino acids selected from the group consisting of Leu, Asp, Ala, Gln, His, Arg, Lys.

In another preferred embodiment, the tyrosine at position 27 and the valine at position 43 are simultaneously mutated to leucine.

In another preferred embodiment, the tyrosine at position 27 and the valine at position 43 are mutated to leucine at the same time, and the D-domain is optionally deleted (threonine on position 62 to glutamic acid on position 67 of SEQ ID NO.: 1).

In another preferred embodiment, the amino acid sequence of the IGF2 mutant is shown in any of SEQ ID NO.: 2-58.

In another preferred embodiment, the IGF2 mutant is a polypeptide having an amino acid sequence as shown in any of SEQ ID NO.: 2-58, an active fragment thereof, or a conservative variant polypeptide thereof.

In another preferred embodiment, except for the mutation (such as Glu at position 12, Phe at position 26, Tyr at position 27, Val at position 43 and corresponding to the mutated sequence that deletes the D-domain), the remaining amino acid sequence of the IGF2 mutant is the same or substantially the same as the sequence as shown in SEQ ID NO.: 1.

In another preferred embodiment, the glutamic acid at position 12 is mutated to glutamine.

In another preferred embodiment, the substantially the same is a difference of at most 50 (preferably 1-20, more preferably 1-10, more preferably 1-5) amino acids, wherein the difference includes amino acid substitution, deletion or addition (e.g. deletion of T at position 62 to E at position 67), and the IGF2 mutant has the activity of inhibiting inflammation.

In another preferred embodiment, the homology with the sequence as shown in SEQ ID NO.: 1 is at least 80%, preferably at least 85% or 90%, more preferably at least 95%, and most preferably at least 98% or 99%.

In another preferred embodiment, the IGF2 protein is derived from human or non-human mammals.

In another preferred embodiment, the IGF2 mutant is selected from the group consisting of:
(a) a polypeptide having the amino acid sequence shown in any of SEQ ID NO.:2-58;
(b) a polypeptide derived from (a), which is formed by the substitution, deletion or addition of one or more (e.g., 2, 3, 4 or 5) amino acid residues in any of the amino acid sequences as shown in SEQ ID NO.: 2-58, and having anti-inflammatory activity.

In another preferred embodiment, the homology between the derived polypeptide and any of the sequences shown in SEQ ID NO.:2-58 is at least 60%, preferably at least 70%, more preferably at least 80%, most preferably at least 90%, such as 95%, 97%, 99%.

In another preferred embodiment, the IGF2 mutant is formed by mutating the wild-type IGF2 protein as shown in SEQ ID NO.: 1.

In another preferred embodiment, the composition or preparation is also used for one or more purposes selected from the group consisting of:
(i) reducing pathogenic mononuclear cell infiltration;
(ii) suppressing the pathogenic phenotype of macrophages;
(iii) reducing the number of macrophages infiltrating;
(iv) improving the concentration of H⁺ in the cytoplasm of macrophages;
(v) improving the metabolic tendency of macrophages to oxidize phosphoric acid;
(vi) Confering macrophages with an immune memory that significantly inhibits inflammation;
(vii) preventing and/or treating autoimmune diseases;
(viii) for a variety of other diseases involving inflammatory responses in which macrophages are involved (such as inflammatory responses associated with the occurrence of liver fibrosis, renal fibrosis, pulmonary fibrosis and other diseases, and inflammatory reactions caused by tissue, organ or cell transplantation, and inflammatory responses associated with the occurrence and metastasis of lung, breast, prostate, liver, pancreatic, melanoma, lymphoma and other cancers, as well as inflammatory responses related to autoimmune diseases);
(ix) promoting implantation of transplanted tissue or cells;
(x) downregulating IGF2R expression on THP1 cells.

In another preferred embodiment, the autoimmune disease is selected from the group consisting of multiple sclerosis, inflammatory bowel disease, autoimmune hepatitis, systemic lupus erythematosus, rheumatoid arthritis, insulin resistance, diabetes, autoimmunity hepatitis, leukoderma, psoriasis, peritonitis, scleroderma, hashimoto thyroiditis, graft-versus-host disease, dermatomyositis, Kawasaki disease, Adult Ear Disease, Ankylosing Spondylitis, Sarcoidosis, enthesitis-related arthritis, Polyarticular Course Juvenile Idiopathic Arthritis, autoimmune pancreatitis, and some autoimmune diseases that are not well defined, and a combination thereof.

In another preferred embodiment, the composition includes a pharmaceutical composition.

In another preferred embodiment, the composition comprises (a) a non-IGF1R-binding substance; and (b) a pharmaceutically acceptable carrier.

In another preferred embodiment, the composition contains 0.001-99 wt%, preferably 0.1-90 wt%, more preferably 1-80 wt% of non-IGF1R-binding substances by total weight of the composition.

In another preferred embodiment, the composition is a liquid preparation or a freeze-dry preparation.

In another preferred embodiment, the composition is an injection.

In a second aspect of the present invention, it provides a cell preparation, comprising:
a phagocyte treated with a non-IGF1R-binding substance.

In another preferred embodiment, the phagocyte is selected from the group consisting of a monocyte, macrophage, monocyte precursor cell, and a combination thereof.

In another preferred embodiment, the non-IGF1R-binding substance is selected from the group consisting of IGF2, an IGF2 mutant, a vector expressing an IGF2 mutant, an antibody, a small molecule compound, and a combination thereof.

In another preferred embodiment, the non-IGF1R-binding substance further includes one or more substances selected from the group consisting of: profibrinolysin (Plasminogen), Serglycin, Cellular repressor of E1A-stimulated genes (CREG), sulfamidase (Sgsh), Phosphorylated β-glucuronidase (Gusb) and other effective anti-inflammatory ligands of IGF2R.

In another preferred embodiment, the cell preparation further includes a non-IGF1R-binding substance.

In another preferred embodiment, the phagocyte has characteristics selected from the group consisting of:
(a) IGF1R is not activated or is less activated than IGF2R, and IGF2R is activated; and/or
(b) oxidative phosphorylation is the main energy source of the phagocyte; and/or
(c) possessing one or more characteristics selected from the group consisting of:
   □ Low expression of IL-1β;
   □ Low expression of TNFα;
   □ Low expression of CXCL10;
   □ High expression of CCL22;
   □ High expression of CLEC7a;
   □ High expression of PD-L1;
   □ High expression of PD-L2;
   □ High expression of IL-10;
   □ High expression of TGFβ;
   □ Increased H⁺ concentration in the cytoplasm;
   □ Nitric oxide is produced, and lactic acid levels decrease.

In another preferred embodiment, the phagocyte is derived from bone marrow, abdominal cavity, peripheral blood, sites of inflammation, and a combination thereof.

In another preferred embodiment, the cell preparation includes a liquid preparation.

In another preferred embodiment, the cell in the cell preparation consist essentially (≥90%, 95%, 96%, 97%, 98%, 99%, 99.5%, 99.9%) or entirely of (a) a phagocyte pretreated with a non-IGF1R-binding substance and (b) optionally a non-IGF1R-binding substance.

In another preferred embodiment, in the composition, the concentration of the phagocyte is 1×10⁴-5×10⁷/ml, preferably 5×10⁴-5×10⁶/ml, more preferably 1×10⁵-1×10⁶/ml.

In another preferred embodiment, the carrier is selected from the group consisting of: an infusion solution carrier and/or an injection carrier, preferably, the carrier is one or more carriers selected from the group consisting of: saline, glucose saline, and a combination thereof.

In another preferred embodiment, the cell preparation further includes other drugs for preventing and/or treating inflammatory diseases.

In another preferred embodiment, the other drugs for preventing and/or treating inflammatory diseases are selected from the group consisting of non-steroidal anti-inflammatory drugs, glucocorticoids, methotrexates, TNFα neutralizing antibodies, TNFR1 antibodies, TNFR2 antibodies, anti-CD20 antibodies, IL-1R antagonists, IL-12 and IL-23p40 neutralizing antibodies, IL-23p19 neutralizing antibodies, IL-17 neutralizing antibodies, IL-17A receptor neutralizing antibodies, and combinations thereof.

In another preferred embodiment, the other drugs for preventing and/or treating autoimmune diseases are selected from the group consisting of non-steroidal anti-inflammatory drugs, glucocorticoids, methotrexates, TNFα neutralizing antibodies, TNFR1 antibodies , TNFR2 antibodies, anti-CD20 antibodies, IL-1R antagonists, IL-12 and IL-23p40 neutralizing antibodies, IL-23p19 neutralizing antibodies, IL-17 neutralizing antibodies, IL-17A receptor neutralizing antibodies, CTLA- 4 fusion proteins, and combinations thereof.

In a third aspect of the present invention, it provides a kit, comprising:
(i) a first container, and the active ingredient (a) a phagocyte treated with a non-IGF1R-binding substance, or a medicament containing the active ingredient (a) contained in the first container;
(ii) an optional second container, and the active ingredient (b) a non-IGF1R-binding substance, or a medicament containing the active ingredient (b), contained in the second container; and
(iii) instructions for preventing and/or treating inflammatory diseases by co-administering active ingredient (a) and active ingredient (b).

In another preferred embodiment, the kit further includes:
(iv) a third container, and the active ingredient (c) other medicaments for preventing and/or treating inflammatory diseases, or a medicament containing the active ingredient (c) contained in the third container.

In another preferred embodiment, the kit further includes:
(v) a fourth container, and the active ingredient (d) other medicaments for preventing and/or treating autoimmune diseases, or a medicament containing other medicaments for preventing and/or treating autoimmune diseases contained in the fourth container.

In another preferred embodiment, the first container, the second container, the optional third container, and the optional fourth container may be the same or different.

In another preferred embodiment, the medicament in the first container is a single preparation containing a phagocyte treated with a non-IGF1R-binding substance.

In another preferred embodiment, the medicament in the second container is a single preparation containing a non-IGF1R-binding substance.

In another preferred embodiment, the third container is a single preparation containing other medicaments for preventing and/or treating inflammatory diseases.

In another preferred embodiment, the fourth container is a single preparation containing other medicaments for preventing and/or treating autoimmune diseases.

In another preferred embodiment, the dosage form of the medicament is an injection.

In another preferred embodiment, the kit further contains instructions, which describe the instructions of the combined administration of active ingredient (a), active ingredient (b), optional (c) and optional (d) thereby (i) preventing and/or treating inflammatory diseases; and/or (ii) preventing and/or treating autoimmune diseases.

In another preferred embodiment, in the preparation containing (b) a non-IGF1R-binding substance, the concentration of the non-IGF1R-binding substance is 0.01 µg-1 mg/kg body weight, preferably 0.1-10 µg /kg body weight, more preferably 0.5-5 µg/kg body weight.

In another preferred embodiment, in the preparation containing (a) a phagocyte treated with a non-IGF1R-binding substance, the concentration of the phagocyte is 0.2^{∗}10⁴-1^{∗}10⁸ cells/kg body weight, preferably 1^{∗}10⁵-5^{∗}10⁷ cells/kg body weight, more preferably 1^{∗}10⁶-5^{∗}10⁶ cells /kg body weight.

In another preferred embodiment, in the preparation containing (c) other medicaments for preventing and/or treating inflammatory diseases, the concentration of the other medicaments for preventing and/or treating inflammatory diseases is 0.0002-40 mg/kg body weight, preferably 0.001-30mg/kg body weight, more preferably 0.02-25mg/kg body weight.

In another preferred embodiment, in the preparation containing (d) other medicaments for preventing and/or treating autoimmune diseases, the concentration of the other medicaments for preventing and/or treating autoimmune diseases is 0.0002-0.1 mg /kg body weight, preferably 0.001-0.08 mg/kg body weight, more preferably 0.002-0.01 mg/kg body weight.

In a fourth aspect of the present invention, it provides a method for screening a medicament, comprising the steps of:
(a) in a test group, adding a test substance to a cell culture system, and observing the binding activity of the test substance to IGF1R and/or IGF2R in the cell of the test group; in the control group, in a control group, no test substance is added to the culture system of the same cell;
wherein, if the binding activity of the test substance to IGF1R in the cell of the test group is decreased; and the binding activity to IGF2R is unchanged or increased, indicating that the test substance is a non-IGF1R-binding IGF2 mutant.

In another preferred embodiment, the method further comprises the steps of: (b) for the non-IGF1R-binding IGF2 mutant obtained in step (a), further testing its binding activity to IGFBP protein; and/or further testing whether its binding to IGFBP protein remains unchanged or slightly increased.

In another preferred embodiment, the method further comprises the steps of:
(c) introducing a positive control, the positive control is an IGF2 protein or IGF2 mutant, and the binding activity to IGF1R and/or IGF2R is compared with binding activity to IGF1R and/or IGF2R of the non-IGF1R-binding IGF2 mutant obtained in step (a).

In another preferred embodiment, the binding activity of the non-IGF1R-binding IGF2 mutant obtained in step (a) to IGF1R and/or IGF2R is comparable to the binding activity of the positive control to IGFlR and/or IGF2R.

In a fourth aspect of the present invention, it provides a method for screening a candidate for preventing and/or treating inflammatory diseases, comprising:
(a) in a test group, adding a test substance to a cell culture system, and observing the effect of the test substance on the expression and/or activity of IGF2R on the cell surface in the cell of the test group; and/or the effect of the test substance on nuclear transport of IGF2R; and/or the effect of the test substance on the redistribution of IGF2R to the cell nucleus; in a control group, the test substance is not added to the culture system of the same cell;
wherein if the test substance of the test group inhibits the expression and/or activity of IGF2R on the cell surface; and/or the test substance increases nuclear transport of IGF2R; and/or the test substance promotes the redistribution of IGF2R to the cell nucleus, indicating that the test substance is a candidate for preventing and/or treating inflammatory diseases.

In another preferred embodiment, the candidate is a candidate with IGF2R selectivity; that is, the candidate is more selective for IGF2R than for IGF1R.

In another preferred embodiment, the candidate selectively and/or preferentially binds to IGF2R, but does not bind or substantially does not bind to IGF1R.

In another preferred embodiment, the cell is a cell cultured in vitro.

In another preferred embodiment, the cell is a phagocyte.

In another preferred embodiment, the phagocyte is selected from the group consisting of a monocyte, macrophage, monocyte precursor cell, and a combination thereof.

In another preferred embodiment, the method is an in vitro method.

In another preferred embodiment, the method is non-diagnostic and non-therapeutic.

In a fifth aspect of the present invention, it provides a method for screening a medicament (or a method for screening a candidate), comprising:
(a) in a test group, adding a test substance to a cell culture system, and observing the effect of the test substance on the binding activity of IGF2 and IGF1R and/or IGF2R in the cell of the test group; in a control group, no test substance is added to the culture system of the same cell;
wherein if the test substance in the cell of the test group inhibits the binding activity of IGF2 and IGF1R; and having no effect on or promoting the binding activity of IGF2 and IGF2R, indicating that the test substance is a non-IGF1R-binding substance ( or a candidate).

In another preferred embodiment, the non-IGF1R-binding substance is selected from the group consisting of an antibody, a small molecule compound, and a combination thereof.

In another preferred embodiment, the method further comprises the steps of:
(b) for the non-IGF1R-binding substance obtained in step (a), further determining its binding activity to IGFBP protein; and/or further testing whether its binding to IGFBP protein remains unchanged or slightly increased.

In another preferred embodiment, the method further comprises the steps of:
(c) introducing a positive control, the positive control is an IGF2 protein or IGF2 mutant, the binding activity to IGF1R and/or IGF2R is compared with the binding activity to IGF1R and/or IGF2R of the non-IGF1R-binding substance obtained in step (a).

In another preferred embodiment, the binding activity of the non-IGF1R-binding substance obtained in step (a) to IGF1R and/or IGF2R is comparable to the binding activity of the positive control to IGF1R and/or IGF2R.

In another preferred embodiment, the cell is a cell cultured in vitro.

In another preferred embodiment, the cell is a phagocyte.

In another preferred embodiment, the phagocyte is selected from the group consisting of a monocyte, macrophage, monocyte precursor cell, and a combination thereof.

In another preferred embodiment, the method is an in vitro method.

In another preferred embodiment, the method is non-diagnostic and non-therapeutic.

In a sixth aspect of the present invention, it provides a method for obtaining a pro-inflammatory sample and an anti-inflammatory sample in vitro, comprising:
in a first set of experiments, treated with low doses of IGF2, thereby obtaining an anti-inflammatory sample; and
in a second set of experiments, treated with high doses of IGF2, thereby obtaining a pro-inflammatory sample.

In another preferred embodiment, in the pro-inflammatory sample, further determining the binding activity of IGF2 to IGF2R.

In another preferred embodiment, in the anti-inflammatory sample, further determining the binding activity of IGF2 to IGF2R.

In a seventh aspect of the present invention, it provides a composition, comprising:
(a) the non-IGF1R-binding IGF2 mutant or candidate obtained according to the method of the fourth or fifth aspect of the present invention; wherein the non-IGF1R-binding IGF2 mutant does not include an IGF2 mutant selected from the group consisting of wild-type IGF2, amino acid fragment at positions 25-91 of wild-type IGF2, IGF2 mutant in which the glutamic acid at position 12 is mutated to Asp, Ala, Gln, His, Arg, or Lys, phenylalanine at position 26 is mutated to IGF2 mutant for Ser, IGF2 mutant with tyrosine mutation at position 27 to Leu, IGF2 mutant with valine at position 43 mutated to Leu, IGF2 mutant with D-domain deleted, and a combination thereof.

In another preferred embodiment, the non-IGF1R-binding IGF2 mutant does not include the IGF2 mutant as shown in any of SEQ ID NO.: 1-2, 16, 18, 20, 22, 24, 26, 28, and 43.

In another preferred embodiment, the amino acid sequence of the non-IGF1R-binding IGF2 mutant is shown in any one of SEQ ID NO.: 3-15, 17, 19, 21, 23, 25, 27, 29-42, 44-58.

In an eighth aspect of the present invention, it provides a method for inhibiting inflammatory activity, comprising the steps of:
In the presence of a non-IGF1R-binding substance (or a candidate with IGF2R selectivity), culturing a phagocyte, thereby inhibiting inflammatory activity.

In another preferred embodiment, the administration concentration of the non-IGF1R-binding substance is 0.0000005-0.0005 mg/ml, preferably 0.000001-0.0001 mg/ml, more preferably 0.000005-0.00005 mg/ml.

In a ninth aspect of the present invention, it provides a method for preventing and/or treating an inflammatory disease, comprising:
(a) administering the cell preparation according to the second aspect of the present invention or the kit according to the third aspect of the present invention or the composition according to the seventh aspect of the present invention to a subject in need.

In another preferred embodiment, the subject includes a non-human mammal and human.

In another preferred embodiment, the administration dosage of the non-IGF1R-binding substance is 0.01 µg-1 mg/kg body weight, preferably 0.1-10 µg/kg body weight, more preferably 0.5-5 µg/kg body weight.

In another preferred embodiment, the administration frequency of the non-IGF1R-binding substance is 1 time/day to 2 times/day.

In another preferred embodiment, the administration includes simultaneous administration or successive administration.

In a tenth aspect of the present invention, it provides a method for preventing and/or treating an autoimmune disease, comprising:
(a) administering the cell preparation according to the second aspect of the present invention or the kit according to the third aspect of the present invention or the composition according to the seventh aspect of the present invention to a subject in need.

In another preferred embodiment, the subject includes a non-human mammal and human.

In another preferred embodiment, the administration dosage of the non-IGF1R-binding substance is 0.01 µg-1 mg/kg body weight, preferably 0.1-10 µg/kg body weight, more preferably 0.5-5 µg/kg body weight.

In another preferred embodiment, the administration frequency of the non-IGF1R-binding substance is 1 time/day to 2 times/day.

In another preferred embodiment, the administration includes simultaneous administration or successive administration.

In an eleventh aspect of the present invention, it provides a use of the cell preparation according to the second aspect of the present invention, the kit according to the third aspect of the present invention, or the composition according to the seventh aspect of the present invention for the preparation of a medicament for preventing and/or treating an inflammatory disease.

It should be understood that, within the scope of the present invention, each technical feature of the present invention described above and in the following (as examples) may be combined with each other to form a new or preferred technical solution, which is not listed here due to space limitations.

### Description of Figure

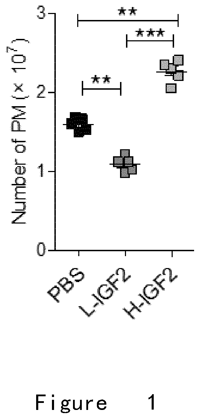

Figure 1 shows that low-dose of IGF2 inhibits peritonitis. Peritonitis mice are treated with low-dose IGF2 (L-IGF2, 5-50 ng IGF2 per mouse) and high-dose IGF2 (H-IGF2, 1000 ng IGF2 per mouse), respectively. Isolation of peritoneal monocytes and macrophages from mice with peritonitis and analysis of the number of peritoneal monocytes and macrophages that can be isolated per mouse. Experiments are repeated three times, and significant differences are performed by student's t-test. ^{∗∗}*P*<0.01; ^{∗∗∗}*P*<0.001.

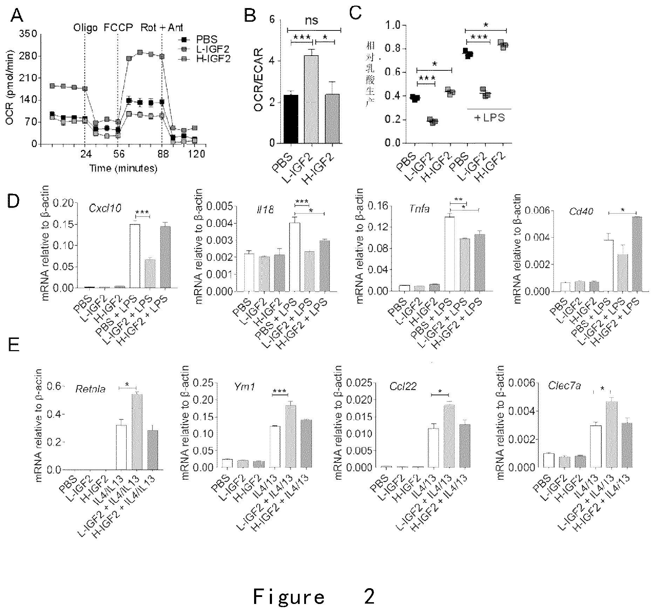

Figure 2 shows that low-dose IGF2 alters macrophage energy metabolism preference and inflammatory factor expression trends and potential. Peritonitis mice are treated with low-dose IGF2 (L-IGF2, 5-50 ng IGF2 per mouse) and high-dose IGF2 (H-IGF2, 1000 ng IGF2 per mouse), respectively. Peritoneal macrophages in peritonitis mice are isolated and analyzed. A-B, Low-dose IGF2 enhances oxidative phosphorylation potential of macrophages, whereas high-dose IGF2 inhibits oxidative phosphorylation. C, Low-dose IGF2 reduces lactate production in macrophages with and without lipopolysaccharide (LPS) stimulation, corresponding to weaker aerobic glycolysis metabolism. D, Low-dose IGF2 inhibits the expression of LPS-induced pro-inflammatory genes (Cxcl10, IL-18, Tnfa, Cd40), E, Low-dose IGF2 promotes expression of IL-4 and IL-13-induced anti-inflammatory repair-related genes (Retnla, Ym1, Ccl22, Clec7a). Experiments are repeated three times, and significant differences are performed by student's t-test. ^{∗}P<0.05; ^{∗∗}P<0.01; ^{∗∗∗}P<0.001.

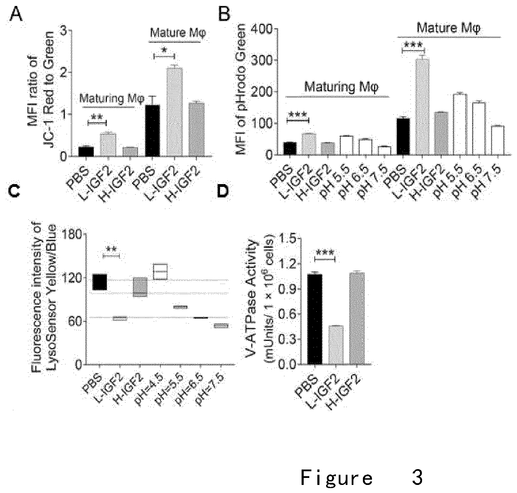

Figure 3 shows that low-dose of IGF2 increase cytoplasmic H⁺ concentrations in monocyte (maturing) and macrophages (mature) and decrease lysosomal H⁺ concentrations. Peritonitis mice are treated with low-dose IGF2 (L-IGF2, 5-50 ng IGF2 per mouse) and high-dose IGF2 (H-IGF2, 1000 ng IGF2 per mouse), respectively. Isolation and analysis of peritoneal monocytes and macrophages in mice with peritonitis. A, Flow cytometry detection of IGF2 regulates the fluorescence intensity of JC1 in peritoneal monocytes and macrophages to evaluate relative mitochondrial membrane potential. B, Cytoplasmic pH of peritoneal monocytes and macrophages is regulated by flow cytometry detection of IGF2. C, Flow cytometry detection of IGF2 regulates the relative pH of lysosomes in peritoneal macrophages. D, IGF2 regulates absolute activity of lysosomal V-type ATPase in peritoneal macrophages.

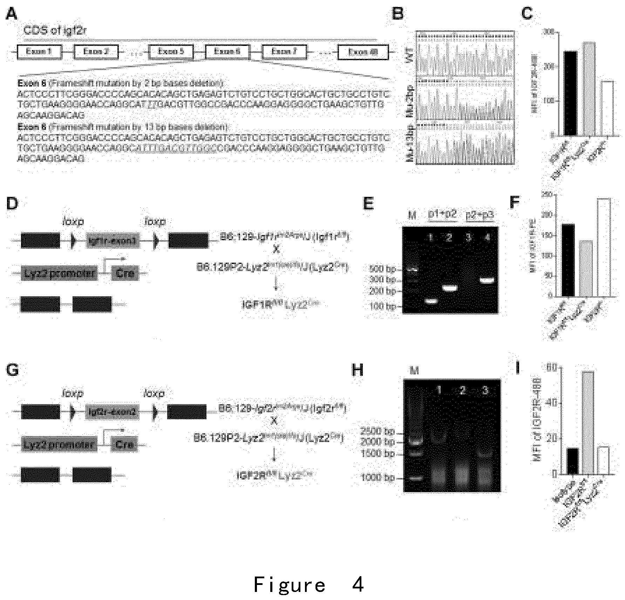

Figure 4 shows the construction of *igf2r* gene-deficient mice, as well as *igf1r* or *igf2r* conditional knockout mice. A-C, Knock down IGF2R gene expression by frameshift mutation caused by 2 bp or 13 bp gene deletion using CRISPR-Cas9 technology (complete knockout of the IGF2R gene results in embryonic lethality in mice), using gene sequencing technology to identify offspring mice, using flow cytometry to identify knockdown efficiency. D-I, by hybriding IGF1R^{*ƒl*/*ƒl*} or IGF2R ^{*ƒl*/*ƒl*} mice and Lyz*^{Cre}* mice, mice with specific knockout of myeloid cells IGF1R or IGF2R are obtained. Knockout efficiencies are identified by PCR and flow cytometric analysis. E, Agarose gel electrophoresis results, lane 1 indicates wild-type mice (124 bp), lane 2 indicates Igf1r^{*ƒl*/*ƒl*} (220 bp), lane 3 has no band, indicating that Igf1r^{*ƒl*/*ƒl*} mice do not have Lyz2*^{Cre}* activity, lane 4 (320 bp) indicates that Igf1r^{*ƒl*/*ƒl*} mice possess Lyz2*^{Cre}* activity. H, Lane 1 indicates wild-type mice (2000 bp), lane 2 indicates Igf2r^{*ƒl*/*ƒl*} mice without Lyz2*^{Cre}* activity (2200 bp).

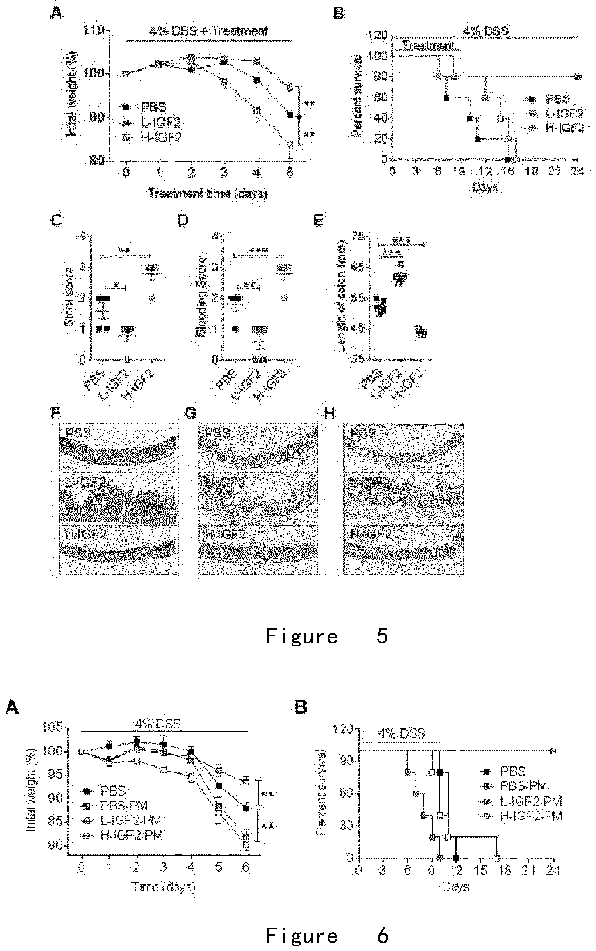

Figure 5 shows that low-dose of IGF2 inhibits dextran sulfate sodium salt (DSS)-induced inflammatory bowel disease. Enteritis mouse model is induced by feeding and drinking 4% DSS solution. A-E, Low-dose IGF2 alleviates DSS-induced inflammatory bowel disease, as demonstrated by significant improvements in body mass index, survival time, faeces score and faeces bleeding score, and colon length. F-H, H&E staining, TUNEL and Ki67 immunohistochemical staining of mouse colon tissue when IGF2 is used to treat DSS-induced colitis mice. Experiments are repeated three times, and significant differences are performed by student's t-test. ^{∗}P<0.05; ^{∗∗}P<0.01; ^{∗∗∗}P<0.001.
Figure 6 shows that low-dose IGF2-treated macrophages can effectively inhibit inflammatory bowel disease. Mice are given 4% DSS solution to induce inflammatory bowel disease mouse model. A-B, the therapeutic effect of low-dose or high-dose IGF2-induced peritoneal monocyte/macrophages (peritoneal macrophages, PM) in DSS-induced inflammatory bowel disease, body weight changes and survival curves are recorded. Experiments are repeated three times, and significant differences are performed by student's t-test. ^{∗∗}P<0.01.

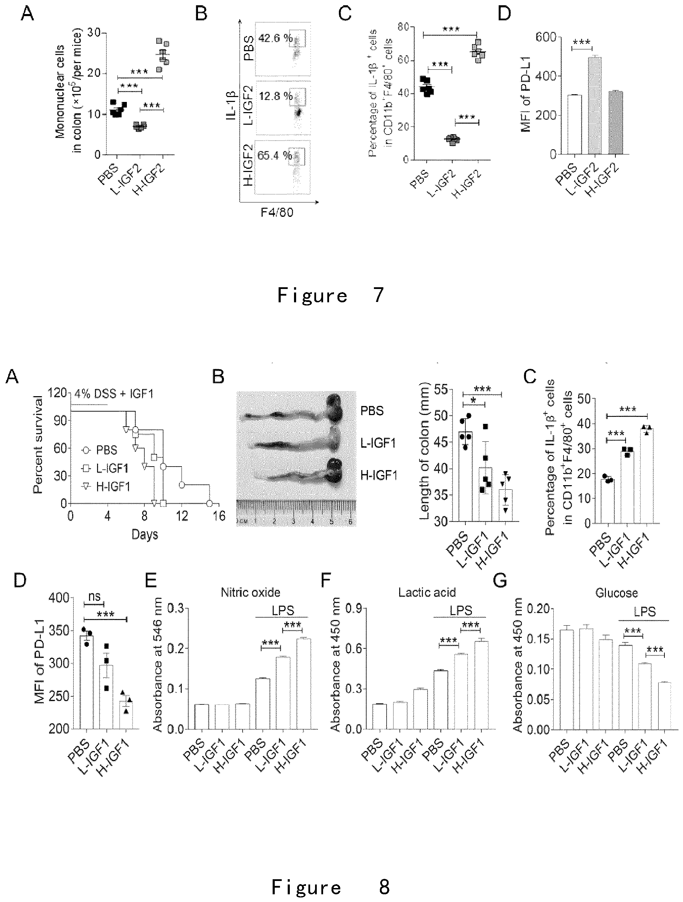

Figure 7 shows that low-dose of IGF2 promote macrophage formation with anti-inflammatory properties. Mice are given 4% DSS solution to induce inflammatory bowel disease mouse model. A, Low-dose IGF2 improves DSS-induced inflammatory bowel disease and reduces mononuclear cell infiltration in colon tissues. B-C, the effect of IGF2 on the expression of interleukin-1β (IL-1β) in infiltrating macrophages (CD11b⁺F4/80⁺) in the colon of mice with inflammatory bowel disease is analyzed. D, Analysis of the effect of IGF2 on PD-L1 expression levels on infiltrating macrophages (CD11b⁺F4/80⁺) in the colon of mice with inflammatory bowel disease. Experiments are repeated three times, and significant differences are performed by student's t-test. ^{∗∗∗}P<0.001.
Figure 8 shows that IGF1 aggravates inflammatory bowel disease progression. Mice are given 4% DSS solution to induce inflammatory bowel disease mouse model. Mice are given 4% DSS solution to induce inflammatory bowel disease mouse model. On day 1, day 3, and day 5 of disease induction, mice with inflammatory bowel disease are injected with low-dose IGF1 (L-IGF1, 50 ng/mouse), high-dose IGF1 (H-IGF1, 1000 ng/mouse) or PBS. A, observing the survival of mice; B, measuring the change in colon length; C, detecting the proportion of IL-1β expressed by macrophages in the colonic lamina propria by flow cytometry; D, detection of PD-L1 expression by macrophages in colonic lamina propria by flow cytometry; E-G, mice with peritonitis are injected with L-IGF1, H-IGF1 or PBS, macrophages are isolated from peritoneal lavage fluid, and the effects of IGF1 on nitric oxide secretion, lactic acid production and glucose consumption in macrophages were analyzed under LPS stimulation.

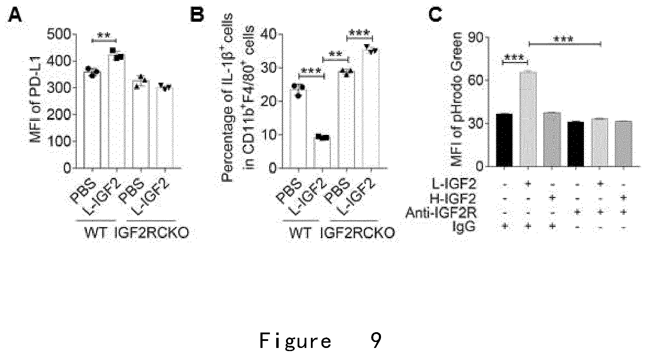

Figure 9 shows that IGF2R performs the regulatory effect of low-dose IGF2 on macrophage anti-inflammatory function. Peritonitis is induced in wild-type (WT) mice and IGF2RCKO (IGF2R^{*ƒl*/*ƒl*}Lyz2*^{Cre}*) mice, and IGF2 (50 ng/mouse) or PBS is injected intraperitoneally, and macrophages are isolated from the peritoneal lavage fluid 48 hours later. A, Flow cytometry analysis of PD-L1 expression in macrophages; B, Flow cytometry analysis of IL-1β expression in macrophages. Mice are given to induce peritonitis, injected with low-dose IGF2 or high-dose IGF2 respectively, and IGF2R antibody is given at the same time, C, H⁺ concentration in the cytoplasm of macrophages is analyzed by flow cytometry.

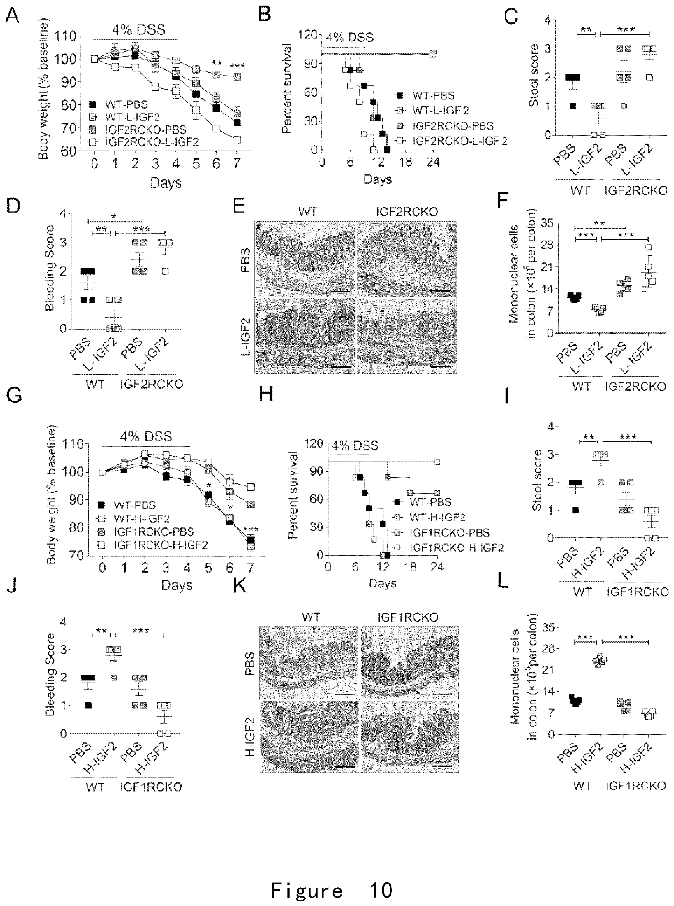

Figure 10 shows that IGF2R performs the anti-inflammatory task of low-dose IGF2. Administering wild type ((Lyz2Cre) mice, IGF2RCKO (IGF2R^{*ƒl*/*ƒl*}Lyz2*^{Cre}*) mice, and IGF1RCKO (IGF1R^{*ƒl*/*ƒl*}Lyz2*^{Cre}*) mice 4% DSS solution to induce a mouse model of enteritis. A-F, Evaluation of the effect of low doses of IGF2 on inflammatory bowel disease induced in IGF2R^{*ƒl*/*ƒl*}Lyz2*^{Cre}* mice and IGF2R^{*ƒl*/*ƒl*} mice. A, weight change of mice; B, survival of mice; C, faeces score; D, scores for blood in faeces; E, histopathological analysis of colon; F, infiltration of mononuclear cells in the colonic lamina propria. G-L, Evaluation of the effect of high doses of IGF2 on inflammatory bowel disease induced in IGF1R^{*ƒl*/*ƒl*}Lyz2*^{Cre}* mice and IGF1R^{*ƒl*/*ƒl*} mice. G, weight change of mice; H, survival of mice; I, faeces score; J, scores for blood in faeces; K, histopathological analysis of colon; L, infiltration of mononuclear cells in the colonic lamina propria. Experiments are repeated three times, and significant differences are performed by student's t-test. ^{∗}P<0.05; ^{∗∗}P<0.01; ^{∗∗∗}P<0.001.

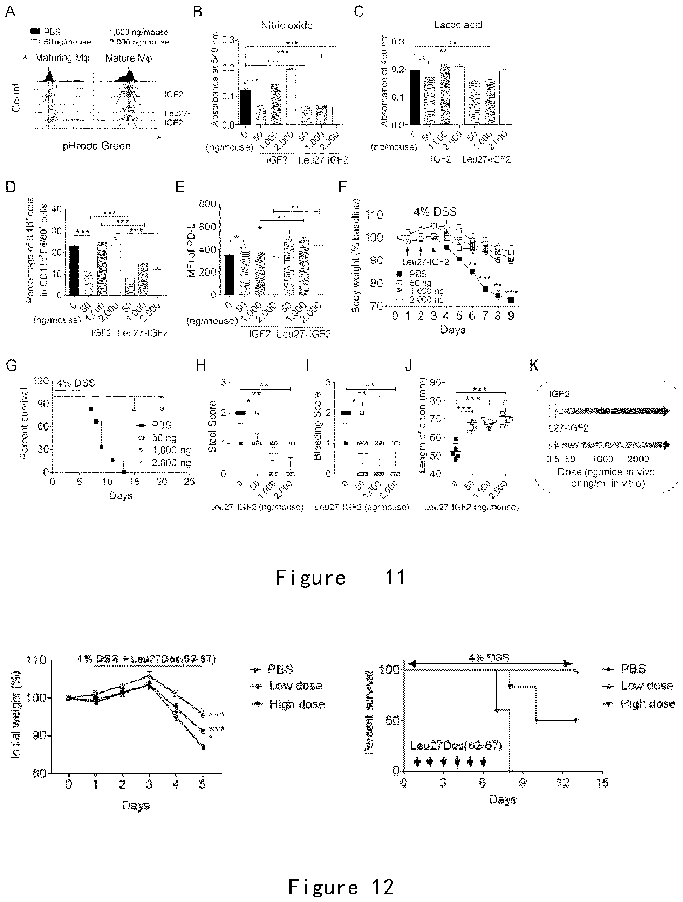

Figure 11 shows that Leu27-IGF2 targeted activation of IGF2R most effectively inhibits peritonitis and inflammatory bowel disease. Peritonitis mice are treated with low-dose IGF2 or Leu27-IGF2 (50 ng IGF2/Leu27-IGF2 per mouse) and high-dose IGF2 or Leu27-IGF2 (1000 ng, or 2000 ng IGF2/Leu27-IGF2 per mouse), respectively. Peritoneal monocytes and macrophages from mice with peritonitis are isolated and analyzed. A, Leu27-IGF2 significantly increases H⁺ concentration in the cytoplasm of macrophages. B-C, The ability of peritoneal macrophages to produce nitric oxide (nitric oxide) and lactate under treatment with different dosages of IGF2 and Leu27-IGF2. D-E, The expression levels of IL-1β and PD-L1 in peritoneal macrophages stimulated by LPS under treatment with different dosages of IGF2 and Leu27-IGF2. Mice are given 4% DSS solution to induce inflammatory bowel disease model. F, Body mass index evaluation of the alleviating effect of Leu27-IGF2 on DSS-induced inflammatory bowel disease. G, Mice survival evaluation of the therapeutic effect of Leu27-IGF2 on DSS-induced inflammatory bowel disease. H-J, faeces score, degree of blood in faeces, colon length evaluation of the therapeutic effect of Leu27-IGF2 on DSS-induced inflammatory bowel disease. K, Schematic illustration of effective thresholds for wild-type IGF2 and Leu27-IGF2 to alleviate Ftg-induced peritonitis and DSS-induced inflammatory bowel disease. Experiments are repeated three times, and significant differences are performed by student's t-test. ^{∗}P<0.05; ^{∗∗}P<0.01; ^{∗∗∗}P<0.001.
Figure 12 shows that Leu27-Des(62-67)-IGF2 inhibits inflammatory bowel disease. Mice are given 4% DSS solution to induce inflammatory bowel disease model, and low-dose and high-dose Leu27-Des(62-67)-IGF2 are injected on the first day of disease induction. A, monitoring the body weight change of mice; B, survival of mice with inflammatory bowel disease, evaluating the therapeutic effect of Leu27-Des(62-67)IGF2 on inflammatory bowel disease. Significant differences are performed by student's t-test. ^{∗}P<0.05; ^{∗∗∗}P<0.001.

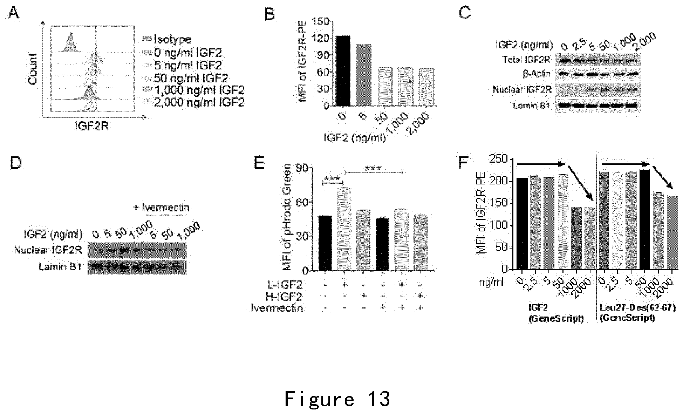

Figure 13 shows that activation of IGF2R promotes nuclear distribution and regulates macrophage cytoplasmic pH. THP1 cells are treated with different dosages of IGF2, A-B, and the amount of IGF2R on the cell membrane surface is detected by flow cytometry. C, Western blotting is used to detect the expression of IGF2R at the overall cellular level and in the nucleus. Bone marrow-derived mononuclear precursor cells treated with different dosages of IGF2 are treated with a protein nuclear transcription inhibitor, ivermectin (5 µ M). After the macrophages matures, D, Western blotting is used to detect the expression of IGF2R at the overall cellular level and in the nucleus. E, Flow cytometry is used to detect the concentration of H⁺ in the cytoplasm of macrophages. THP1 cells were treated with different dosages of IGF2 or Leu27-Des(62-67)-IGF2, F, and the expression of IGF2R on the cell membrane surface is detected by flow cytometry. Significant differences are performed by student's t-test. ^{∗∗∗}P<0.001.

### DETAILED DESCRIPTION

After extensive and in-depth research, the present inventors have unexpectedly discovered for the first time that non-IGF1R-binding substances can significantly (a) prevent and/or treat inflammatory diseases; and/or (b) prevent and/or treat autoimmune diseases. On this basis, the present inventors have completed the present invention.

As used herein, wild-type IGF2, an amino acid fragment at positions 25-91 of wild-type IGF2, IGF2 mutants with glutamic acid at position 12 mutated to Asp, Ala, Gln, His, Arg, or Lys, IGF2 mutants with phenylalanine at position 26 mutated to Ser, IGF2 mutants with tyrosine at position 27 mutated to Leu, IGF2 mutants with valine at position 43 mutated to Leu, and IGF2 mutants with D-domain deleted are all amino acid truncations, mutations and/or deletions on the basis of SEQ ID NO.: 1.

### IGF2 protein and a mutant thereof

Insulin like growth factor 2 (IGF2) belongs to the insulin-like growth factor family, involved with other families in the regulation of cell proliferation, differentiation, metabolism, aging and death, affecting the development, growth, health and disease of organisms^{1,2}. IGF2 receptors include IGF1R and IGF2R, which have the highest affinity with IGF2R, and can bind to IGF1R next, and hardly bind to insulin receptors².

IGF2 and IGF1 have a high degree of homology in amino acid sequence, and there is a 62% overlapping sequence. Therefore, IGF2 can often reflect the biological effects that IGF1 can activate². Current studies suggest that the main biological function of IGF2 is dependent on the activation of the tyrosine kinase that activates IGF1R (insulin like growth factor 1 receptor), and has no positive correlation with IGF2R (insulin like growth factor 2 receptor) and IR. From an evolutionary point of view, IGF1R shares gene homology with IR, and the signaling pathways activated downstream of IGF1R and IR are very similar. However, the gene of IGF2R is completely different from the source of IGF1R or IR gene. Therefore, IGF2R is often considered to have the opposite biological function of IGF1R and IR. IGF2R acts as a degradation receptor of IGF2, and the activation of IGF1R by IGF2 is inhibited by internalizing and degrading IGF2.

About 90% of IGF2R is distributed in the intracellular Golgi apparatus, and the remaining 10% of that is distributed in the outer cell membrane³. IGF2R is not the only receptor of IGF2, nor is IGF2 the only ligand of IGF2R. Its ligands also include mannose-6-phosphate. In existing reports, the main function of IGF2R is to transport its ligands to lysosomes for protein processing or degradation⁴. In addition, IGF2R on the cell membrane can be cleaved in a soluble form into the blood and other fluid environments, where it can effectively bind to IGF2 and inhibit IGF2 function^{4,5}. A preliminary study of the present invention has found that IGF2 can inhibit animal models of multiple sclerosis, ulcerative colitis and other diseases, but its specific regulatory mechanism is still unclear.

Specifically, the present invention relates to an IGF2 protein and a variant thereof. In a preferred embodiment of the present invention, the amino acid sequence of the IGF2 protein is shown in SEQ ID NO.:1. The IGF2 protein or the vector expressing the IGF2 protein of the present invention can (a) prevent and/or treat inflammatory diseases.

The IGF2 protein of the present invention or the vector expressing the IGF2 protein can also be used for one or more purposes selected from the group consisting of:
(i) reducing pathogenic mononuclear cell infiltration;
(ii) suppressing the pathogenic phenotype of macrophages;
(iii) reducing the number of macrophages infiltrating;
(iv) increasing the concentration of H⁺ in the cytoplasm of macrophages;
(v) increasing the metabolic tendency of macrophages to oxidize phosphoric acid;
(vi) confering macrophages with an immune memory that significantly inhibits inflammation;
(vii) preventing and/or treating autoimmune diseases;
(viii) for a variety of other diseases involving inflammatory response in which macrophages are involved (such as inflammatory response related to the occurrence of liver fibrosis, renal fibrosis, pulmonary fibrosis, etc., inflammatory response to tissue, organ, or cell transplantation, inflammatory responses associated with the occurrence and metastasis of cancers such as lung cancer, breast cancer, prostate cancer, liver cancer, pancreatic cancer, melanoma, lymphoma, and inflammatory responses associated with autoimmune diseases);
(ix) facilitating the implantation of transplanted tissue or cells;
(x) downregulation of IGF2R expression on THP1 cells.

The present invention also includes polypeptides or proteins with the same or similar functions that have 50% or more (preferably more than 60%, more than 70%, more than 80%, more preferably more than 90%, more preferably more than 95%, most preferably more than 98%, such as 99%) homology with the sequence as shown in SEQ ID NO.: 1 of the present invention.

Wherein, SEQ ID NO.: 1 is human IGF2 protein (AYRPSETLCGGELVDTLQFVCGDRGFYFSRPASRVSRRSRGIVEECCFRSCD LALLETYCATPAKSE).

The "same or similar function" mainly refers to: "(i) reducing pathogenic mononuclear cell infiltration; and/or (ii) suppressing the pathogenic phenotype of macrophages; and/or (iii) reducing the number of macrophages infiltrating; and/or (iv) increasing the concentration of H⁺ in the cytoplasm of macrophages; and/or (v) increasing the metabolic tendency of macrophages to oxidize phosphoric acid; and/or (vi) confering macrophages with an immune memory that significantly inhibits inflammation; and/or (vii) preventing and/or treating autoimmune diseases; and/or (viii) for a variety of other diseases involving inflammatory response in which macrophages are involved (such as inflammatory response related to the occurrence of liver fibrosis, renal fibrosis, pulmonary fibrosis, etc., inflammatory response to tissue, organ, or cell transplantation, inflammatory responses associated with the occurrence and metastasis of cancers such as lung cancer, breast cancer, prostate cancer, liver cancer, pancreatic cancer, melanoma, lymphoma, and inflammatory responses associated with autoimmune diseases); and/or (ix) facilitating the implantation of transplanted tissue or cells; and/or (x) downregulation of IGF2R expression on THP1 cells".

The protein of the present invention can be a recombinant protein, a natural protein, or a synthetic protein. The proteins of the invention may be naturally purified products, or chemically synthesized products, or produced using recombinant techniques from prokaryotic or eukaryotic hosts (e.g., bacterial, yeast, higher plant, insect and mammalian cells). Depending on the host used in the recombinant production protocol, the proteins of the invention may be glycosylated or may be non-glycosylated. The proteins of the invention may or may not also include an initial methionine residue.

The present invention also includes IGF2 protein fragments and analogs having IGF2 protein activity. As used herein, the terms "fragment" and "analog" refer to proteins that retain substantially the same biological function or activity of the native IGF2 protein of the invention.

In a preferred embodiment, the mutant of the IGF2 protein of the present invention is mutated at the tyrosine corresponding to the position 27 of SEQ ID NO.: 1 (AYRPSETLCGGELVDTLQFVCGDRGFYFSRPASRVSRRSRGIVEECCFRSCD LALLETYCATPAKSE) of the wild-type IGF2 protein and the mutant of the IGF2 protein of the present invention can significantly (a) prevent and/or treat inflammatory diseases; and/or (b) prevent and/or treat autoimmune diseases.

Preferably, the tyrosine at position 27 is mutated to leucine.

Preferably, the tyrosine at position 27 is mutated to leucine, and the D-domain is optionally deleted (threonine at position 62 to glutamic acid at position 67 of SEQ ID NO.: 1).

In a preferred embodiment, the IGF2 mutant is a polypeptide having an amino acid sequence shown in any of SEQ ID NO.: 2-58, an active fragment thereof, or a conservative variant polypeptide thereof.

It should be understood that the amino acid numbering in the mutants of the present invention is based on SEQ ID NO.: 1, and when a mutant has 80% or more homology with the sequence shown in SEQ ID NO.: 1, the amino acid numbering of the mutants may have a misalignment with respect to the amino acid numbering of SEQ ID NO.: 1, such as 1-5 positions are shifted to the N-terminus or C-terminus of amino acids, and using conventional sequence alignment techniques in the art, those skilled in the art will generally understand that such dislocations are within a reasonable range, and should not be due to the misplacement of amino acid numbering, those with homology of 80% (e.g., 90%, 95%, 98%) having the same or similar activity that can significantly (a) prevent and/or treat inflammatory diseases; and/or; (i) reducing pathogenic mononuclear cell infiltration; and/or (ii) suppressing the pathogenic phenotype of macrophages; and/or (iii) reducing the number of macrophages infiltrating; and/or (iv) increasing the concentration of H⁺ in the cytoplasm of macrophages; and/or (v) increasing the metabolic tendency of macrophages to oxidize phosphoric acid; and/or (vi) confering macrophages with an immune memory that significantly inhibits inflammation; and/or (vii) preventing and/or treating autoimmune diseases; and/or (viii) for a variety of other diseases involving inflammatory response in which macrophages are involved (such as inflammatory response related to the occurrence of liver fibrosis, renal fibrosis, pulmonary fibrosis, etc., inflammatory response to tissue, organ, or cell transplantation, inflammatory responses associated with the occurrence and metastasis of cancers such as lung cancer, breast cancer, prostate cancer, liver cancer, pancreatic cancer, melanoma, lymphoma, and inflammatory responses associated with autoimmune diseases); and/or (ix) facilitating the implantation of transplanted tissue or cells; and/or (x) downregulation of IGF2R expression on THP1 cells are not within the scope of the mutants of the present invention.

The mutein fragments, derivatives or analogs of the present invention may be (i) muteins having one or more conservative or non-conservative amino acid residues (preferably conservative amino acid residues) substituted, and such substituted amino acid residues may or may not be encoded by the genetic code, or (ii) a mutein with a substitution group in one or more amino acid residues, or (iii) a mutein formed by fusion of the mature mutein with another compound (such as a compound that prolongs the half-life of the mutein, such as polyethylene glycol), or (iv) a mutein formed by fusing an additional amino acid sequence to this mutein sequence (such as a leader sequence or a secretion sequence or the sequence or pro-protein sequence used to purify this mutein, or a fusion protein with an antigenic IgG fragment). These fragments, derivatives and analogs are well known to those skilled in the art in light of the teachings herein. In the present invention, conservatively substituted amino acids are preferably produced by amino acid substitutions according to Table 1.

**Table I**

| initial residue | representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also includes polypeptides or proteins with the same or similar functions that have 50% or more (preferably more than 60%, more than 70%, more than 80%, more preferably more than 90%, more preferably more than 95%, most preferably more than 98%, such as 99%) homology with the native IGF2 protein of the present invention. In the protein variant, several (usually 1-60, preferably 1-30, more preferably 1-20, most preferably 1-10) substitutions, deletions or additions of at least one amino acid may result in a derived sequence, as well as the addition of one or several (usually within 20, preferably within 10, more preferably within 5) amino acids to the C-terminal and/or N-terminal. For example, in the protein, when substituted with amino acids with similar or close properties, the function of the protein is usually not changed, and the addition of one or several amino acids at the C-terminus and/or \-terminus usually does not change the function of the protein. The present invention includes the difference between the natural IGF2 protein analog and the natural IGF2 protein, which can be the difference in amino acid sequence, or the difference in modified form that does not affect the sequence, or both. Analogs of these proteins include natural or induced genetic variants. Induced variants can be obtained by a variety of techniques, such as random mutagenesis by radiation or exposure to mutagens, site-directed mutagenesis or other known molecular biology techniques. Analogs also include analogs with residues other than natural L-amino acids (e.g., D-amino acids), as well as analogs with non-naturally occurring or synthetic amino acids (e.g., beta, gamma-amino acids). It should be understood that the proteins of the present invention are not limited to the representative proteins exemplified above.

Modified (usually without altering the primary structure) forms include chemically derivatized forms of the protein in vivo or in vitro such as acetate or carboxylation. Modifications also include glycosylation, such as glycosylation modifications such as those are carried out in protein synthesis and processing. This modification can be accomplished by exposing the protein to enzymes that perform glycosylation, such as mammalian glycosylases or deglycosylases. Modified forms also include sequences with phosphorylated amino acid residues (eg, phosphotyrosine, phosphoserine, phosphothreonine). In addition, the muteins of the present invention can also be modified. Modified (usually without altering the primary structure) forms include chemically derivatized forms of the protein in vivo or in vitro such as acetate or carboxylation. Modifications also include glycosylation, such as glycosylation modifications such as those are carried out in protein synthesis and processing. This modification can be accomplished by exposing the protein to enzymes that perform glycosylation, such as mammalian glycosylases or deglycosylases. Modified forms also include sequences with phosphorylated amino acid residues (eg, phosphotyrosine, phosphoserine, phosphothreonine). Also included are mutant proteins that have been modified to increase their resistance to proteolysis or to optimize solubility.

The present invention also provides a polynucleotide sequence encoding the IGF2 protein. The polynucleotides of the present invention may be in the form of DNA or RNA. DNA forms include: DNA, genomic DNA or synthetic DNA, DNA can be single-stranded or double-stranded. Polynucleotides encoding mature polypeptides include: coding sequences encoding only the mature polypeptide; coding sequences and various additional coding sequences for the mature polypeptide; coding sequences (and optional additional coding sequences) for the mature polypeptide, and non-coding sequences. The term "polynucleotide encoding a polypeptide" may include a polynucleotide encoding the polypeptide or a polynucleotide that also includes additional coding and/or non-coding sequences. The present invention also relates to variants of the above-mentioned polynucleotides, which encode fragments, analogs and derivatives of polypeptides having the same amino acid sequence as the present invention. Variants of this polynucleotide can be naturally occurring allelic variants or non-naturally occurring variants. These nucleotide variants include substitution variants, deletion variants, and insertion variants. As known in the art, an allelic variant is an alternative form of a polynucleotide, which may be a substitution, deletion or insertion of one or more nucleotides that does not substantially alter the function of the encoded polypeptide.

Based on the nucleotide sequences described herein, those skilled in the art can readily prepare the encoding nucleic acids of the present invention by various known methods. These methods are such as but not limited to: PCR, artificial DNA synthesis, etc. For specific methods, please refer to J. Sambrook, " Guidelines for molecular cloning experiments". As an embodiment of the present invention, the coding nucleic acid sequence of the present invention can be constructed by the method of segmental synthesis of nucleotide sequences followed by overlap extension PCR.

The present invention also relates to polynucleotides that hybridize to the above-mentioned sequences and have at least 50%, preferably at least 70%, more preferably at least 80% identity between the two sequences. The present invention particularly relates to polynucleotides that are hybridizable under strict conditions (or stringent conditions) to the polynucleotides of the present invention. In the present invention, "strict conditions" refer to: (1) hybridization and elution at lower ionic strength and higher temperature, such as 0.2×SSC, 0.1% SDS, 60°C; or (2) a denaturant is added during hybridization, such as 50% (v/v) formamide, 0.1% calf serum/0.1% Ficoll, 42°C, etc. or (3) Hybridization occurs only when the identity between the two sequences is at least 90% or more, more preferably 95% or more. The proteins and polynucleotides of the present invention are preferably provided in isolated form, more preferably, purified to homogeneity.

The full-length sequence of the polynucleotide of the present invention can usually be obtained by PCR amplification method, recombinant method or artificial synthesis method. For the PCR amplification method, primers can be designed according to the relevant nucleotide sequences disclosed in the present invention, especially the open reading frame sequences, and the relevant sequences can be obtained by amplification by using a commercially available cDNA library or a cDNA library prepared by conventional methods known to those skilled in the art as a template. When the sequence is long, it is often necessary to perform two or more PCR amplifications, and then splicing the amplified fragments together in the correct order.

Once the relevant sequences have been obtained, recombinant methods can be used to obtain the relevant sequences in bulk. This is usually done by cloning it into a vector, transferring it into a cell, and isolating the relevant sequence from the propagated host cell by conventional methods.

In addition, synthetic methods can also be used to synthesize the relevant sequences, especially when the fragment length is short. Often, fragments of very long sequences are obtained by synthesizing multiple small fragments followed by ligation.

At present, the DNA sequences encoding the proteins of the present invention (or fragments thereof, or derivatives thereof) can be obtained entirely by chemical synthesis. This DNA sequence can then be introduced into various existing DNA molecules (or e.g., vectors) and cells known in the art. In addition, mutations can also be introduced into the protein sequences of the invention by chemical synthesis.

Methods of amplifying DNA/RNA using PCR techniques are preferred for obtaining the polynucleotides of the present invention. In particular, when it is difficult to obtain a full-length cDNA from the library, the RACE method (RACE-rapid amplification of cDNA ends) can be preferably used, and the primers for PCR can be appropriately selected according to the sequence information of the present invention disclosed herein, and can be synthesized by conventional methods. Amplified DNA/RNA fragments can be isolated and purified by conventional methods such as by gel electrophoresis.

### Expression vector

The present invention also relates to vectors comprising the polynucleotides of the present invention, as well as host cells genetically engineered with the vectors of the present invention or the coding sequences of the muteins of the present invention, and methods for producing the polypeptides of the present invention by recombinant techniques.

The polynucleotide sequences of the present invention can be used to express or produce recombinant muteins by conventional recombinant DNA techniques. Generally there are the following steps:
(1) Transform or transduce a suitable host cell with the polynucleotide (or variant) of the present invention encoding the mutein of the present invention, or with a recombinant expression vector containing the polynucleotide;
(2). Host cells cultured in a suitable medium;
(3). Separation and purification of proteins from culture medium or cells.

In the present invention, the polynucleotide sequence encoding the mutein can be inserted into a recombinant expression vector. The term "recombinant expression vector" refers to bacterial plasmids, bacteriophages, yeast plasmids, plant cell viruses, mammalian cell viruses such as adenoviruses, retroviruses, or other vectors well known in the art. Any plasmids and vectors can be used as long as they are replicable and stable in the host. An important feature of expression vectors is that they typically contain an origin of replication, a promoter, marker gene and translational control element.

Methods well known to those skilled in the art can be used to construct expression vectors containing the DNA sequences encoding the muteins of the invention and appropriate transcriptional/translational control signals. These methods include in vitro recombinant DNA technology, DNA synthesis technology, in vivo recombinant technology, and the like. The DNA sequence can be operably linked to an appropriate promoter in an expression vector to direct mRNA synthesis. Representative examples of these promoters are: the lac or trp promoter of E. coli; the λ phage PL promoter; eukaryotic promoters include the CMV immediate early promoter, the HSV thymidine kinase promoter, the early and late SV40 promoters, the reverse The LTRs of retrovirus and some other known promoters that control the expression of genes in prokaryotic or eukaryotic cells or their viruses. Expression vectors also include a ribosome binding site for translation initiation and a transcription terminator.

In addition, the expression vector preferably contains one or more selectable marker genes to provide phenotypic traits for selection of transformed host cells, such as dihydrofolate reductase for eukaryotic cell culture, neomycin resistance, and green fluorescent protein (GFP), or for tetracycline or ampicillin resistance in E. coli.

Vectors comprising the appropriate DNA sequences described above, together with appropriate promoter or control sequences, can be used to transform appropriate host cells so that they can express the protein.

Host cells can be prokaryotic cells, such as bacterial cells; or lower eukaryotic cells, such as yeast cells; or higher eukaryotic cells, such as mammalian cells. Representative examples are: Escherichia coli, Streptomyces; bacterial cells of Salmonella typhimurium; fungal cells such as yeast, plant cells (e.g., ginseng cells).

When the polynucleotides of the present invention are expressed in higher eukaryotic cells, transcription will be enhanced if an enhancer sequence is inserted into the vector. Enhancers are cis-acting elements of DNA, usually about 10 to 300 base pairs in length, that act on a promoter to enhance transcription of a gene. Illustrative examples include the SV40 enhancer of 100 to 270 base pairs on the late side of the replication origin, the polyoma enhancer on the late side of the replication origin, and the adenovirus enhancer, etc.

It will be clear to those of ordinary skill in the art how to select appropriate vectors, promoters, enhancers and host cells.

Transformation of host cells with recombinant DNA can be performed using conventional techniques well known to those skilled in the art. When the host is a prokaryotic organism such as E. coli, competent cells capable of uptake of DNA can be harvested after exponential growth phase and treated with the CaCl2 method using procedures well known in the art. Another way is to use MgCl2. If desired, transformation can also be performed by electroporation. When the host is a eukaryotic organism, the following DNA transfection methods can be used: calcium phosphate co-precipitation method, conventional mechanical methods such as microinjection, electroporation, liposome packaging and the like.

The obtained transformants can be cultured by conventional methods to express the polypeptides encoded by the genes of the present invention. The medium used in the culture can be selected from various conventional media depending on the host cells used. Cultivation is carried out under conditions suitable for growth of the host cells. After the host cells have grown to an appropriate cell density, the selected promoter is induced by a suitable method (eg, temperature switching or chemical induction), and the cells are cultured for an additional period of time.

The recombinant polypeptide in the above method can be expressed intracellularly, or on the cell membrane, or secreted outside the cell. If desired, recombinant proteins can be isolated and purified by various isolation methods utilizing their physical, chemical and other properties. These methods are well known to those skilled in the art. Examples of such methods include, but are not limited to: conventional renaturation treatment, treatment with protein precipitants (salting-out method), centrifugation, osmotic disruption, ultratreatment, ultracentrifugation, molecular sieve chromatography (gel filtration), adsorption chromatography, ion exchange chromatography, high performance liquid chromatography (HPLC) and various other liquid chromatography techniques and combinations of these methods.

### non-IGF1R-binding substances

As used herein, the terms "non-IGFIR binding", "IGF2R selective" are used interchangeably to refer to the substance being more selective for IGF2R than for IGF1R, i.e. selectively and/or preferentially binds to IGF2R and does not bind or substantially does not bind to IGF1R.

In the present invention, non-IGF1R-binding substances include substances with higher selectivity or affinity for IGF2R than IGF1R, as well as substances that do not activate IGF1R (such as retinoic acid and other non-IGF1R ligands) that can efficiently activate IGF2R. In a preferred embodiment, the non-IGF1R-binding substance has a higher affinity or selectivity for IGF2R than its affinity or selectivity for IGF1R. In a preferred embodiment, the non-IGF1R-binding substance is selected from the group consisting of an IGF2 mutant, a vector expressing an IGF2 mutant, an antibody, a small molecule compound, or a combination thereof.

In a preferred embodiment, the non-IGF1R-binding substance further comprises one or more substances selected from the group consisting of: Plasminogen, serglycin, Cellular repressor of E1A-stimulated genes (CREG), Sulfamidase (Sgsh), Phosphorylated β-glucuronidase (Gusb) and other IGF2R effective anti-inflammatory ligands.

In the present invention, it has been unexpectedly discovered for the first time that non-IGF1R-binding substances can significantly (a) prevent and/or treat inflammatory diseases; and/or (i) reducing pathogenic mononuclear cell infiltration; and/or (ii) suppressing the pathogenic phenotype of macrophages; and/or (iii) reducing the number of macrophages infiltrating; and/or (iv) increasing the concentration of H⁺ in the cytoplasm of macrophages; and/or (v) increasing the metabolic tendency of macrophages to oxidize phosphoric acid; and/or (vi) confering macrophages with an immune memory that significantly inhibits inflammation; and/or (vii) preventing and/or treating autoimmune diseases; and/or (viii) for a variety of other diseases involving inflammatory response in which macrophages are involved (such as inflammatory response related to the occurrence of liver fibrosis, renal fibrosis, pulmonary fibrosis, etc., inflammatory response to tissue, organ, or cell transplantation, inflammatory responses associated with the occurrence and metastasis of cancers such as lung cancer, breast cancer, prostate cancer, liver cancer, pancreatic cancer, melanoma, lymphoma, and inflammatory responses associated with autoimmune diseases); and/or (ix) facilitating the implantation of transplanted tissue or cells; and/or (x) downregulation of IGF2R expression on THP1 cells.

### Compound pharmaceutical compositions and kits

The present invention provides a compound pharmaceutical composition comprising active ingredients (a) a phagocyte treated with a non-IGF1R-binding substance; (b) an optional non-IGF1R-binding substance; and (c) a pharmaceutically acceptable carrier. Such carriers include, but are not limited to, saline, buffers, dextrose, water, glycerol, ethanol, powders, and combinations thereof. The drug formulation should match the mode of administration. The pharmaceutical composition of the present invention can be prepared in the form of injection, for example, prepared by conventional methods with physiological saline or an aqueous solution containing glucose and other adjuvants. Pharmaceutical compositions, such as tablets and capsules, can be prepared by conventional methods. Pharmaceutical compositions such as injections, solutions, tablets and capsules are preferably manufactured under sterile conditions. The pharmaceutical combination of the present invention may also be formulated as a powder for aerosol inhalation. The dosage form of the pharmaceutical composition of the present invention is an injection. The amount of active ingredient administered is a therapeutically effective amount. The pharmaceutical preparation of the present invention can also be made into a sustained release preparation. The pharmaceutical composition of the present invention is preferably an injection preparation. In addition, the pharmaceutical compositions of the present invention may also be used with other therapeutic agents and the pharmaceutical composition of the present invention may also include additional components selected from the group consisting of: (a) drugs for preventing and/or treating inflammatory diseases; and/or (b) components for the prevention and/or treatment of autoimmune diseases.

The effective amount of the active ingredient of the present invention may vary with the mode of administration, the severity of the disease to be treated, and the like. Selection of the preferred effective amount can be determined by one of ordinary skill in the art based on various factors (eg, through clinical trials). The factors include, but are not limited to: the pharmacokinetic parameters of the active ingredient such as bioavailability, metabolism, half-life, etc.; the severity of the disease the patient is being treated, the weight of the patient, the immune status of the patient, route of the administration, etc. Typically, satisfactory effects are obtained when the active ingredient of the present invention is administered at a dosage of about 0.01 µg-1 mg/kg body weight, preferably 0.1-10 µg/kg body weight, more preferably 0.5-5 µg/kg body weight per day. The optimal dosage will be adjusted appropriately according to the course and condition of the disease, eg, several divided doses may be administered daily or the dose may be proportionally reduced as dictated by the exigencies of the therapeutic situation.

The pharmaceutically acceptable carriers of the present invention include (but are not limited to): water, saline, liposomes, lipids, proteins, protein-antibody conjugates, peptides, cellulose, nanogels, or its combination. The choice of carrier should match the mode of administration, as is well known to those of ordinary skill in the art.

The present invention also provides a kit for (a) preventing and/or treating inflammatory diseases; and/or (i) reducing pathogenic mononuclear cell infiltration; and/or (ii) suppressing the pathogenic phenotype of macrophages; and/or (iii) reducing the number of macrophages infiltrating; and/or (iv) increasing the concentration of H⁺ in the cytoplasm of macrophages; and/or (v) increasing the metabolic tendency of macrophages to oxidize phosphoric acid; and/or (vi) confering macrophages with an immune memory that significantly inhibits inflammation; and/or (vii) preventing and/or treating autoimmune diseases; and/or (viii) for a variety of other diseases involving inflammatory response in which macrophages are involved (such as inflammatory response related to the occurrence of liver fibrosis, renal fibrosis, pulmonary fibrosis, etc., inflammatory response to tissue, organ, or cell transplantation, inflammatory responses associated with the occurrence and metastasis of cancers such as lung cancer, breast cancer, prostate cancer, liver cancer, pancreatic cancer, melanoma, lymphoma, and inflammatory responses associated with autoimmune diseases); and/or (ix) facilitating the implantation of transplanted tissue or cells; and/or (x) downregulation of IGF2R expression on THP1 cells, the kit contains:
(i) a first container, and the active ingredient (a) a phagocyte treated with a non-IGF1R-binding substance, or a medicament containing the active ingredient (a) contained in the first container;
(ii) an optional second container, and the active ingredient (b) a non-IGF1R-binding substance, or a medicament containing the active ingredient (b), contained in the second container; and
(iii) instructions for administering the active ingredient (a) for (a) preventing and/or treating an inflammatory disease; and/or (i) reducing pathogenic mononuclear cell infiltration; and/or (ii) suppressing the pathogenic phenotype of macrophages; and/or (iii) reducing the number of macrophages infiltrating; and/or
(iv) increasing the concentration of H⁺ in the cytoplasm of macrophages; and/or (v) increasing the metabolic tendency of macrophages to oxidize phosphoric acid; and/or (vi) conferring macrophages with an immune memory that significantly inhibits inflammation; and/or (vii) preventing and/or treating autoimmune diseases; and/or (viii) for a variety of other diseases involving inflammatory response in which macrophages are involved (such as inflammatory response related to the occurrence of liver fibrosis, renal fibrosis, pulmonary fibrosis, etc., inflammatory response to tissue, organ, or cell transplantation, inflammatory responses associated with the occurrence and metastasis of cancers such as lung cancer, breast cancer, prostate cancer, liver cancer, pancreatic cancer, melanoma, lymphoma, and inflammatory responses associated with autoimmune diseases); and/or (ix) facilitating the implantation of transplanted tissue or cells; and/or (x) downregulation of IGF2R expression on THP1 cells.

The pharmaceutical compositions and kits of the present invention are suitable for use in (a) preventing and/or treating inflammatory diseases; and/or (i) reducing infiltration of pathogenic mononuclear cells in the colon; and/or(ii) suppressing the pathogenic phenotype of macrophages; and/or (iii) reducing the number of macrophages infiltrating; and/or (iv) increasing the concentration of H⁺ in the cytoplasm of macrophages; and/or (v) increasing the metabolic tendency of macrophages to oxidize phosphoric acid; and/or (vi) conferring macrophages with an immune memory that significantly inhibits inflammation; and/or (vii) preventing and/or treating autoimmune diseases; and/or (viii) for a variety of other diseases involving inflammatory response in which macrophages are involved (such as inflammatory response related to the occurrence of liver fibrosis, renal fibrosis, pulmonary fibrosis, etc., inflammatory response to tissue, organ, or cell transplantation, inflammatory responses associated with the occurrence and metastasis of cancers such as lung cancer, breast cancer, prostate cancer, liver cancer, pancreatic cancer, melanoma, lymphoma, and inflammatory responses associated with autoimmune diseases); and/or (ix) facilitating the implantation of transplanted tissue or cells; and/or (x) downregulation of IGF2R expression on THP1 cells.

The preparation of the present invention can be taken three times a day to once every ten days, or once every ten days in a sustained-release manner. The preferred way is to take it once a day, because it is easy for the patient to adhere to, thereby significantly improving the patient's compliance with the medication.

When taken, the total daily dose generally applied in the vast majority of cases should be lower than (or a few cases equal to or slightly larger than) the usual daily dose of each single drug. Of course, the effective dose of the active ingredient employed may vary with the mode of administration, the severity of the disease to be treated, and the like.

### Treatment method

The present invention also provides a method of the two active ingredients of the present invention or the corresponding medicaments for (a) preventing and/or treating inflammatory diseases; and/or (i) reducing pathogenic mononuclear cell infiltration; and/or (ii) suppressing the pathogenic phenotype of macrophages; and/or (iii) reducing the number of macrophages infiltrating; and/or (iv) increasing the concentration of H⁺ in the cytoplasm of macrophages; and/or (v) increasing the metabolic tendency of macrophages to oxidize phosphoric acid; and/or (vi) conferring macrophages with an immune memory that significantly inhibits inflammation; and/or (vii) preventing and/or treating autoimmune diseases; and/or (viii) for a variety of other diseases involving inflammatory response in which macrophages are involved (such as inflammatory response related to the occurrence of liver fibrosis, renal fibrosis, pulmonary fibrosis, etc., inflammatory response to tissue, organ, or cell transplantation, inflammatory responses associated with the occurrence and metastasis of cancers such as lung cancer, breast cancer, prostate cancer, liver cancer, pancreatic cancer, melanoma, lymphoma, and inflammatory responses associated with autoimmune diseases); and/or (ix) facilitating the implantation of transplanted tissue or cells; and/or (x) downregulation of IGF2R expression on THP1 cells, comprising administering to a mammal an effective amount of an active ingredient (a) a phagocyte treated with a non-IGF1R-binding substance, or administering a pharmaceutical composition containing the active ingredient (a); and (b) a non-IGF1R-binding substance, or a pharmaceutical composition containing the active ingredient (b).

When the two active ingredients of the present invention are used for the above purposes, they can be mixed with one or more pharmaceutically acceptable carriers or excipients, such as solvents, diluents, etc., and can be administered orally in the form of tablets, pills, capsules, dispersible powders, granules or suspensions (containing e.g. about 0.05-5% suspending agent), syrups (containing e.g. about 10-50% sugar), and elixirs (containing about 20-50% ethanol), Alternatively, parenteral administration is in the form of sterile injectable solutions or suspensions (containing about 0.05-5% suspending agent in an isotonic medium). For example, these pharmaceutical preparations may contain about 0.01-99%, more preferably about 0.1-90% by weight of the active ingredient in admixture with the carrier.

The two active ingredients or pharmaceutical compositions of the present invention may be administered by conventional routes including, but not limited to: intramuscular, intraperitoneal, intravenous, subcutaneous, intradermal, oral, intratumoral or topical administration. Preferred routes of administration include oral, intramuscular or intravenous administration.

From the standpoint of ease of administration, preferred pharmaceutical compositions are liquid compositions, especially injections.

In addition, the two active ingredients or medicaments of the present invention can also be used in combination with other components or drugs for (a) prevention and/or treatment of inflammatory diseases; and/or and/or (i) reducing pathogenic mononuclear cell infiltration; and/or (ii) suppressing the pathogenic phenotype of macrophages; and/or (iii) reducing the number of macrophages infiltrating; and/or (iv) increasing the concentration of H⁺ in the cytoplasm of macrophages; and/or (v) increasing the metabolic tendency of macrophages to oxidize phosphoric acid; and/or (vi) conferring macrophages with an immune memory that significantly inhibits inflammation; and/or (vii) preventing and/or treating autoimmune diseases; and/or (viii) for a variety of other diseases involving inflammatory response in which macrophages are involved (such as inflammatory response related to the occurrence of liver fibrosis, renal fibrosis, pulmonary fibrosis, etc., inflammatory response to tissue, organ, or cell transplantation, inflammatory responses associated with the occurrence and metastasis of cancers such as lung cancer, breast cancer, prostate cancer, liver cancer, pancreatic cancer, melanoma, lymphoma, and inflammatory responses associated with autoimmune diseases); and/or (ix) facilitating the implantation of transplanted tissue or cells; and/or (x) downregulation of IGF2R expression on THP1 cells (NSAIDs, glucocorticoids, methotrexate, TNFα neutralizing antibodies, TNFR1 antibodies, TNFR2 antibodies, anti-CD20 antibodies, IL -1R antagonist, IL-12 and IL-23p40 neutralizing antibody, IL-23p19 neutralizing antibody, IL-17 neutralizing antibody, IL-17A receptor neutralizing antibody, CTLA-4 fusion protein).

### The main advantages of the present invention include:

(1) The present invention is the first to discover that non-IGF1R-binding substances can significantly (a) prevent and/or treat inflammatory diseases; and/or (i) reducing pathogenic mononuclear cell infiltration; and/or (ii) suppressing the pathogenic phenotype of macrophages; and/or (iii) reducing the number of macrophages infiltrating; and/or (iv) increasing the concentration of H⁺ in the cytoplasm of macrophages; and/or (v) increasing the metabolic tendency of macrophages to oxidize phosphoric acid; and/or (vi) conferring macrophages with an immune memory that significantly inhibits inflammation; and/or (vii) preventing and/or treating autoimmune diseases; and/or (viii) for a variety of other diseases involving inflammatory response in which macrophages are involved (such as inflammatory response related to the occurrence of liver fibrosis, renal fibrosis, pulmonary fibrosis, etc., inflammatory response to tissue, organ, or cell transplantation, inflammatory responses associated with the occurrence and metastasis of cancers such as lung cancer, breast cancer, prostate cancer, liver cancer, pancreatic cancer, melanoma, lymphoma, and inflammatory responses associated with autoimmune diseases); and/or (ix) facilitating the implantation of transplanted tissue or cells; and/or (x) downregulation of IGF2R expression on THP1 cells.
(2) The present inventors have discovered for the first time that insulin-like growth factor 2 (IGF2) confers anti-inflammatory immune memory on induced macrophages. This property is dependent on the IGF2 receptor (IGF2R). Functionally blocking IGF2R, IGF2 no longer confers immune memory to macrophages against inflammation.
(3) The present invention finds for the first time that low doses of IGF2 can induce non-classical mitochondrial dynamics and endow macrophages with immune memory against inflammation, while high doses of IGF2 can activate the IGF1 receptor (IGF1R), inhibit the effect of IGF2R, and promotes the utilization of aerobic glycolytic metabolism by monocytes and macrophages for their pro-inflammatory capacity.
(4) The present invention finds for the first time that IGF2 mutants with low affinity to IGF1R can specifically activate IGF2R, and relieve autoimmune and inflammatory diseases such as peritonitis and inflammatory bowel disease to the greatest extent. These findings suggest that targeted activation of IGF2R can determine the long-term immunosuppressive memory of macrophages, and IGF2R is an ideal target for the treatment of macrophage-involved inflammation-related diseases
(5) The present invention finds for the first time that the targeted activation of IGF2R in monocytes inhibits inflammatory bowel disease.
(6) The present invention finds for the first time that IGF2 treatment of inflammatory bowel disease depends on macrophages.
(7) The present invention finds for the first time that IGF2 is involved in a variety of other inflammatory response diseases in which macrophages are involved.

The present invention will be further described below in conjunction with specific embodiments. It should be understood that these examples are only used to illustrate the present invention and not to limit the scope of the present invention. The experimental method of unreceipted specific conditions in the following examples, usually according to normal conditions such as people such as Sambrook, molecular cloning: the conditions described in the laboratory manual (New York:Cold Spring Harbor Laboratory Press, 1989), or according to the manufacturer the proposed conditions. Percentages and parts are by weight unless otherwise indicated.

Unless otherwise specified, all materials and reagents used in the examples are commercially available products.

### Materials and Methods

### 1 Reagent

There are many experimental reagents involved in this research. In order to reduce the number of words and save the layout, the reagents will be listed in order according to the different brands of reagents.

Recombinant human insulin-like growth factor-2 (Recombinant human IGF2, 292-G2-250) and recombinant murine macrophage colony-stimulating factor (Recombinant mouse M-CSF protein, 416-ML-050) were purchased from R&D Systems.

Human IGF2 with leucine point mutation at position 27 (Human Leu27-IGF2, TU100) was purchased from GroPep Bioreagents.

Human IGF2 with leucine point mutation at position 27 (Human Leu27-IGF2, TU100) deletes the D domain and is synthesized in the laboratory.

### 2 Construction and identification of experimental mice

In IGF2R^{+/-} mice, Crisper-Cas9 technology was used to knock out 2 bp or 13 bp in exon 6 of the *igf2r* gene, resulting in a frameshift mutation. Since IGF2R-deficient homozygotes are embryonic lethal, we passaged and identified IGF2R^{+/-} heterozygous mice. The primers used for identification were:
Primer 1, 5'-CGTGAAGTCTGTCTATGGAAGAGACTGGACC-3' (SEQ ID NO.:59),
Primer 2, 5'-CATTACCAAGCTCACACTCCCTTCTCTTCTCTATT-3' (SEQ ID NO.: 60),

The genotype result determination method will be reflected in the experimental results section.

In Igf2r^{*ƒl*/*ƒl*} mice, loxp sequences were inserted on both sides of the 2nd exon of the *igf2r* gene, and the primers used for identification were:
Primer 1, 5'-CCCCGGTGTTGAGGTTGATAGATA-3' (SEQ ID NO.:61),
Primer 2, 5'-CAATTTAGGGCTGAAGCGATGGAT-3' (SEQ ID NO.: 62),

The genotype result determination method will be reflected in the experimental results section.

The primers used for the identification of *Igƒ1r*^{*ƒl*/*ƒl*} mice were:
Primer 1, 5'-CTTCCCAGCTTGCTACTCTAGG-3' (SEQ ID NO.:63),
Primer 2, 5'-CAGGCTTGCAATGAGACATGGG-3' (SEQ ID NO.:64),
Primer 3, 5'-TGAGACGTAGCGAGATTGCTGTA-3' (SEQ ID NO.:65)

The genotype result determination method will be reflected in the experimental results section.

The primers used for *Lyz2^{Cre}* mouse identification were:
Primer 1, 5'-CCCAGAAATGCCAGATTACG-3' (SEQ ID NO.: 66),
Primer 2, 5'-CTTGGGCTGCCAGAATTTCTC-3' (SEQ ID NO.: 67),
Primer 3, 5'-TTACAGTCGGCCAGGCTGAC-3' (SEQ ID NO.:68)

The genotype result determination method will be reflected in the experimental results section.

### 3. Peritonitis mouse model

A 5% (mass/volume) thioglycolate (Ftg) medium aqueous solution was prepared, sterilized at 121°C under high temperature and high pressure for 30 minutes and cooled to room temperature for later use. Check the physical properties of the thioglycolate medium before use. The viscosity should be such that no bubbles appear when shaking. The color of the medium should change from blue-green at the initial configuration to brown when shaken.

Select C57BL/6 female mice of the same age within 9-12 weeks for the experiment. Each mouse was slowly injected with 2 ml of thioglycolate medium into the abdominal cavity, and 24 hours later, IGF2 or other drug treatment was administered, and administered once at 24 hours and 48 hours after thioglycolate injection, respectively, the mice were euthanized at 72 hours, and peritoneal macrophages were isolated.

### 4 Peritoneal macrophage isolation

Immerse euthanized (Ftg) mice for 72 hours with 75% ethanol for several minutes, tear open the abdominal fur along the direction perpendicular to the head and tail of the mice, and draw 10 ml of pre-cooled phosphate buffer ( ) with a syringe. Phosphate buffer is injected vigorously and rapidly (to help blow the adherent cells down) into the abdominal cavity of the mouse, the right index finger and ring finger clamp the mouse tail, gently hold the mouse, shake vigorously up and down three to five times, aspirate the cell lavage fluid slowly with a syringe, avoiding puncturing the intestine and contaminating the cells during this process, and avoiding aspirating adipose tissue and clogging the needle. The peritoneal macrophage lavage fluid needs to be stored at four degrees, and the cells are easily adsorbed to the wall of the centrifuge tube at room temperature.

### 5 Models of Inflammatory Bowel Disease

Pre-prepared 16% (mass/volume) aqueous solution of dextran sulfate sodium salt (DSS), filter-sterilized with a 0.45 µM filter tip, and passed into the SPF barrier. The 16% DSS solution was diluted 4-fold with regular drinking water to a final concentration of 4%. Select 9-12-week-old female mice to be fed 4% DSS solution for different days, and the termination date depends on the purpose of the experiment. In the initial experiment to verify the therapeutic effect of IGF2, 4% DSS solution was continuously fed and terminated after the survival curve statistics were completed; in subsequent experiments, 4% DSS solution was usually fed continuously for 5 days, and the specific drinking time of different batches of experiments will be reflected in the experimental results.

For the clinical scoring of inflammatory bowel disease, the following criteria were used. "Stool scores" and "Bleeding scores" were evaluated after feeding the mice with 4% DSS solution for 4 days. The criteria for "Stool scores" are: 0 points, normal stool form; 1 point, semi-formed stools and no anus attachment; 2 points, semi-formed stools with anal attachment; 3 points, liquid stools attached to the anus. The criteria for "Bleeding scores": 0 points, negative for fecal occult blood test paper; 1 point, positive for fecal occult blood test paper; 2 points, visible blood stains on the cage; 3 points, hemoproctia, with red blood spots in the anus.

### 6 Isolation of Colon infiltrating lymphocyte

Euthanize mice induced with 4% DSS for 4-5 days, dissect the mice, obtain colon tissue, and place it in phosphate buffer pre-cooled at 4°C to wash away the blood stains on the colon surface. The colon was cut longitudinally and the feces were carefully washed away in 4°C pre-cooled phosphate buffer. Use 1640 medium containing 200 µg/ml DNaseI and 1 mg/ml type VIII collagenase, add DTT before digestion, shake at 200 rpm for digestion for 60 minutes, carefully remove the basal layer of the colon, cut it into small pieces, and digested again, filter through a 70 µm cell strainers, and centrifuge the filtrate at 400 g for 5 minutes to obtain a single-cell precipitation.

Prepare 40% and 80% Percoll separation solution, resuspend the single cell precipitation with 2ml volume of 40% Percoll, and add 80% Percoll separation solution to the bottom of the centrifuge tube at the lower layer of 40% Percoll. Properly control the pressure on the pipette to prevent the two layers of separation liquid from being mixed and turbid. Adjust the temperature of the centrifuge to 25°C, set the lift acceleration gradient to 0, and centrifuge at 2000 rpm for 25 minutes. Cells in the middle layer of 40% and 80% Percoll separation medium were collected, washed at least twice with 10 ml of 4°C pre-cooled phosphate buffer to completely remove Percoll, and finally pure colon-derived mononuclear cells were obtained.

### 7 Flow Cytometry

Because monocytes and macrophages express relatively high levels of the receptor for IgG, CD16/32, for antibody staining of monocytes and macrophages, it is necessary to block cells in advance with a non-fluorescein-conjugated CD16/32 antibody (antibody : Phosphate buffer = 1:50 dilution of antibody, 100 µl antibody working solution/1×10⁷ cells, incubated at room temperature for 30 minutes). Because macrophages are easily adherent and indigestible, ultra-low adsorption culture plates should be selected to hold cells for macrophage staining.

Cell membrane surface protein staining. For the cells blocked with CD16/32 antibody, dilute the antibody with phosphate buffer at a ratio of 1:100, 100 µl staining working solution/1×10⁷ cells, washed with phosphate buffer after staining for 30 minutes at room temperature, and after centrifugation, resuspend in 200 µl of phosphate buffer and test on machine or fix with 1% neutral formaldehyde.

Intracellular and intranuclear protein staining. Intracellular proteins and intranuclear proteins need to be stained after the completion of cell membrane surface protein staining. For intracellular proteins, use the Cell Fixation and Permeabilization Kit (Fixation/Permeabilization Solution and Perm/Wash Buffer) for permeabilization and post-fixation staining; for nuclear proteins, use the Cell Fixation/Permeabilization Kit (Foxp3/Transcription Factor Staining Buffer Set) for fixation and staining after breaking the membrane.

### 8 Extracellular flux analysis (hippocampal assay)

The culture medium of peritoneal macrophages or THP1 cells (human monocytes) was replaced with Seahorse special medium (XF medium) supplemented with 10 mM glucose, 2 mM glutamine and 2 mM pyruvic acid. Oxygen consumption rate measurement experiment and extracellular acid production rate assay were performed with or without the addition of the following stimulus conditions: 1 µM oligomycin, 0.75 mM FCCP, 100 nM rotenone, and 1 µM antimycin, measured using an extracellular flux analyzer (Agilent) assay.

### 9 Real-time quantitative PCR

Total cellular RNA was extracted using the Tiangen Bio-Cell/Bacterial RNA Isolation Kit, typically 300 ng/ml of RNA in a 30 µL volume can be obtained per sample. All RNAs were reverse transcribed into cDNA using Takara reverse transcription kit (PCR conditions: 37°C for 15 minutes, 85°C for inactivation for 5 seconds, and the temperature was lowered to 4°C for storage). cDNA can be stored at -20°C for several days and degradation occurs over time.

Before using the cDNA, 10-fold dilution of cDNA with double distilled water and the real-time quantitative PCR system is 10 µL, including 5 µL SYBR, 4 µL cDNA, and 1 µL primers. After spotting the samples in a 384-well plate, centrifuged at 2500 rpm for 2 minutes at room temperature. If centrifuged at 4°C during this step, water mist will form on the membrane. The real-time quantitative PCR conditions are: Step 1, 95°C for 10 minutes, Step 2, 95°C for 15 seconds, Step 3, 60°C for 1 minute, Step 4, 95°C for 15 seconds, Step 5, 60°C for 1 minute, Step 6, 95°C for 15 seconds, in which Step 2 and Step 3 are cycled for 40 times.

The primers involved in the real-time quantitative PCR experiment are as follows:
TNFa-F: 5'-CCTGTAGCCCACGTCGTAG-3' (SEQ ID NO.:69),
TNFa-R: 5'-GGGAGTAGACAAGGTACAACCC-3' (SEQ ID NO.: 70);
IL-1b-F: 5'-GCAACTGTTCCTGAACTCAACT-3' (SEQ ID NO.:71),
IL-1b-R: 5'-ATCTTTTGGGGTCCGTCAACT-3' (SEQ ID NO.:72);
Il18-F: 5'-GACTCTTGCGGTCAACTTCAAGG-3' (SEQ ID NO.: 81)
Il18-R: 5'-CAGGCTGTCTTTTGTCAACGA' (SEQ ID NO.: 82);
Cxcl10-F: 5'-CCAAGTGCTGCCGTCATTTTC-3' (SEQ ID NO.:73),
Cxcl10-R: 5'-GGCTCGCAGGGATGATTTCAA-3' (SEQ ID NO.: 74);
Retnla-F: 5'-CCAATCCAGCTAACTATCCCTCC-3' (SEQ ID NO.:75),
Retnla-R: 5'-ACCCAGTAGCAGTCATCCCA-3' (SEQ ID NO.: 76);
CCL22-F: 5'-AGGTCCCTATGGTGCCAATGT-3' (SEQ ID NO.:77),
CCL22-R: 5'-CGGCAGGATTTTGAGGTCCA-3' (SEQ ID NO.: 78);
Clec7a-F: 5'-GACTTCAGCACTCAAGACATCC-3' (SEQ ID NO.:79),
Clec7a-R: 5'-TTGTGTCGCCAAAATGCTAGG-3' (SEQ ID NO.: 80);
Ym1-F: 5'-CAGGTCTGCAATTCTTCTGAA-3' (SEQ ID NO.: 83),
Ym1-R: 5'-GTCTTGCTCATGTGTGTAAGTGA-3' (SEQ ID NO.: 84);
10 H&E staining

Dehydration and embedding: The colon tissue at the same position was taken and fixed in 4% paraformaldehyde solution for 24 hours, and then dehydrated and embedded in the following steps. 70% ethanol, soaking overnight; 80% ethanol, soaking for 2 hours; 85% ethanol, soaking for 1.5 hours; 90% ethanol, soaking for 1 hour; 95% ethanol, soaking for 1 hour; 100% ethanol, soaking for 30 minutes, repeated once ; Xylene, soaking for 5 minutes, repeated once; 60 °C liquid paraffin, soaking for 1 hour, repeated once; use a paraffin embedding machine to complete the paraffin embedding.

Staining of sections: slice at a thickness of 3 µm, extend the paraffin tissue sections in a water bath at 56°C for a few seconds, absorb the paraffin tissue sections with a glass slide, bake the sections in an oven at 60°C overnight, and then stain as follows. Xylene, soak for 5 minutes, repeated once; 100% ethanol, soak for 5 minutes, repeated once; 95% ethanol, soak for 5 minutes, repeated once; 70% ethanol, soak for 5 minutes; distilled water, soak for 5 minutes; Haematoxylin, dyeing for 5 minutes; rinsing with running water for an appropriate time; ammonium hydroxide, soaking for an appropriate time; rinsing with running water for an appropriate time; eosin, staining for 1 minute; 95% ethanol, soaking for 1 minute; 100% ethanol, soaking for 1 minute, repeated once. Immerse in xylene for 1 minute; repeated once; mounted with mounting medium.

### 11 Immunohistochemistry

Antigen renaturation of paraffin sections: drying the paraffin sections in a 60°C oven for two hours; immersing the paraffin sections with pH 7.4 phosphate buffer three times for three minutes each time; The dewaxed and hydrated paraffin sections were placed in boiling pH=6.0 citrate buffer and boiled for ten minutes; after natural cooling in running water, rinsed with distilled water twice for three minutes each time; rinsed with pH 7.4 phosphate buffer twice for three minutes each time. Add one drop of 3% hydrogen peroxide to each paraffin section and incubated at room temperature for ten minutes.

Antigen-antibody reaction: drop the primary antibody against Ki67 on the paraffin section, incubated overnight at 4°C; washed four times with pH 7.4 phosphate-Tween buffer, three minutes each time; The horseradish peroxidase-conjugated secondary antibody was added dropwise to the paraffin section, and incubated at room temperature for 60 minutes; washed 4 times with phosphate-Tween buffer pH 7.4, 3 minutes each; DBA stain was washed off after appropriate time of DBA staining; Counterstain paraffin sections with hematoxylin dye to determine appropriate time based on tissue type; rinsed with tap water to remove hematoxylin; differentiated with hydrochloric acid alcohol and reverse blue with ammonia water until hematoxylin stained to light blue; dehydrated and mounted.

TUNEL immunohistochemical experiments were performed according to the experimental method provided by the In situ cell death detection kit-POD kit (Roche, TUN11684817).

### 12 Nitric oxide detection

Aspirate 50 µL of the culture medium of macrophages stimulated by lipopolysaccharide for 24 hours, add it to a 96-well flat-bottom light-transmitting plate, and set 3 sub-wells for each sample. Add 50 µL of Griess reagent to each well and mixed well, incubated in the dark at room temperature for 15 minutes, and measure the absorbance at 540 nm wavelength on a microplate reader. For quantitative detection experiments, 100 mM NaNO₂ standard stock solution can be pre-prepared, and the gradient dilution is 0, 5, 10, 20, 30, 40, 50, 100 µM of different concentrations of the standard, adding an equal volume of Griess reagent, a standard curve was drawn after incubation and plate reading under the same conditions. Detection of the accumulation of nitric oxide in the supernatant of the medium can reflect the activity of inducible nitric oxide synthase (iNOS) in macrophages, and speculation of macrophage energy metabolism type and inflammatory phenotype.

### 13 Lactic acid detection

The lactate production capacity of IGF2-preprogrammed peritoneal monocytes and macrophages was detected using a lactate assay kit. Experiments were performed according to the experimental method provided by Lactate Colorimetric Assay Kit II (BioVision, K627-100). Detection of the accumulation of lactate in the supernatant of the medium can reflect the type of energy metabolism and inflammatory phenotype of macrophages.

### 14 Determination of glucose concentration

The glucose-consuming capacity of IGF2-preprogrammed peritoneal monocytes and macrophages was detected using a glucose detection kit. It was performed according to the experimental method provided by Glucose Colorimetric Assay Kit II (BioVision, K686-100). The rate of glucose consumption can reflect the energy metabolism type and inflammatory phenotype of macrophages.

### 15 JC1 staining

JC1 staining belongs to live cell staining, pre-configured with JC1 staining working solution (JC1 stock solution: JC1 staining buffer: double distilled water: complete medium = 1:10:89:100 volume ratio configuration), 100µl staining working solution/1 × 10⁷ cells were stained in a 37°C incubator for 30 minutes and detected immediately on the machine. Collect fluorescence information under the FL1 and FL2 channels of the BD calibur flow cytometer. According to the characteristics of JC1 live cell staining: when the mitochondrial membrane potential is high, JC1 oligomerizes to the mitochondrial outer membrane, showing red fluorescence; when the mitochondrial membrane potential decreases, JC1 distributes in the cytoplasm in the form of monomers, showing green fluorescence, so that by comparing the intensities of red fluorescence and green fluorescence, the mitochondrial membrane potential at the single cell level can be evaluated.

### 16 Cytoplasmic and lysosomal pH assays

In live cell staining, the pH and glucose concentration of the cell's environment can have an effect on the intracellular pH. Configure pH staining working solution (pH staining storage solution: activation solution: phosphate buffer = 1:10:89 volume ratio configuration), 100 µl staining working solution/1 × 10⁷ cells, immediately after staining in a 37°C incubator for 30 minutes, the assay was performed on the machine. For cytoplasmic pH detection, use pHrodo Green AM dye, and for lysosomal pH staining, use LysoSensor Green DND-189 or LysoSensor Yellow/Blue DND-160 dye. For cytoplasmic and organelle pH quantification, use the pH detection standard kit (Intracellular pH Calibration Buffer Kit) in combination.

### 17 Lysosome isolation and ATPase activity detection

Lysosomes of IGF2-treated peritoneal monocytes and macrophages were isolated, and experiments were performed according to the experimental method provided by Lysosome Isolation Kit (Sigma-Aldrich, LYSISO1-1KT). The isolated lysosomes will be used for V-type ATPase activity detection experiments. The ATPase activity of the lysosomes isolated above was detected using an ATPase activity detection kit, which represented the activity of the lysosomal V-type ATPase. The experiment was carried out according to the experimental method provided by the ATPase Activity Assay Kit (BioVision, K417).

### 18 Statistical analysis of data

Data were presented in the form of mean or mean ± S.E.M. or S.D., and were plotted using Graphpad software and analyzed for significant differences. In this study, the two-tailed nonparametric Student's t test was used to analyze the significance of differences between the two groups of data. ns, indicates not significant difference; ^{∗}P<0.05; ^{∗∗}P<0.01; ^{∗∗∗}P<0.001, P<0.05 was considered to be significantly different.

### Example 1 Low-dose IGF2 inhibits peritonitis.

Peritonitis mice were treated with low-dose IGF2 (L-IGF2, 5-50 ng IGF2 per mouse) and high-dose IGF2 (H-IGF2, 1000 ng IGF2 per mouse), respectively. Peritoneal monocytes and macrophages from mice with peritonitis were isolated and analyzed.

The results showe that compared with the control group (PBS) and the high-dose IGF2-treated group, low-dose IGF2 can significantly inhibit the infiltration of macrophages in the peritoneal cavity of peritonitis mice (Figure 1), indicating that low-dose IGF2 can inhibit inflammation in peritonitis mice..

### Example 2 The anti-inflammatory macrophages under the regulation of low-dose IGF2 are dominated by the metabolic preference of oxidative phosphorylation.

Peritonitis mice were treated with low-dose IGF2 (L-IGF2, 5-50 ng IGF2 per mouse) and high-dose IGF2 (H-IGF2, 1000 ng IGF2 per mouse) respectively to isolate peritonitis mouse peritoneal macrophages and analyze the metabolic state of the cells. Hippocampal experiments (Seahorse technology) is the gold standard for studying cellular energy metabolism preferences. Through seahorse experiments, we have found that the maximum oxygen consumption rate (OCR) of peritoneal macrophages under the regulation of low-dose IGF2 is significantly higher than that of the control group, the maximum oxygen consumption rate of peritoneal macrophages derived from high-dose IGF2 regulation is lower than that of the control group (Fig. 2A). So that low-dose IGF2 increases the oxygen consumption potential of macrophages, whereas high-dose IGF2 reverses this potential.

In addition, we also measured the maximum extracellular acidification rate (ECAR) of the three groups of cells by seahorse experiments, and have found that the maximum extracellular acid production rate of macrophages under the regulation of low-dose IGF2 is significantly lower than that of the PBS control group and high-dose IGF2 group. These results are better reflected in the ratio of maximal oxygen consumption rate to maximal extracellular acid production rate (OCR:ECAR) (Fig. 2B). Macrophages under the regulation of low-dose IGF2 show higher levels of OCR:ECAR value, indicating that this group of macrophages tends to obtain energy by oxidative phosphorylation (OXPHOS) as the main way, while the macrophages in the control group and high-dose IGF2 group are more inclined to choose aerobic glycolysis to generate energy for cells.

Lactic acid is an intermediate metabolite of aerobic glycolysis. During the process of aerobic glycolysis, excess lactic acid will be secreted by cells to the outside of the cell. Therefore, detecting and comparing the accumulated amount of lactic acid in the supernatant of the cell culture medium is an efficient way to measure the level of aerobic glycolysis performed by cells. We used lipopolysaccharide (LPS) to stimulate these isolated peritoneal macrophages again, and detected the lactic acid content in the supernatant of the medium 24 hours later. It is found that the lactate accumulation in the medium supernatant of peritoneal macrophages in the low-dose IGF2-treated group is significantly lower than that in the PBS and high-dose IGF2-treated groups (Fig. 2C). The above studies have shown that low-dose IGF2 has conferred stronger oxidative phosphorylation capacitation potential in mouse peritoneal macrophages, whereas high-dose IGF2 has reversed this property and even increased the cells' anaerobic glycolytic capacitation preference. Macrophages with oxidative phosphorylation as the main capacitative method often have the characteristics of replacing activated macrophages, reflecting the phenotype of anti-inflammatory. In order to verify the immunophenotype of peritoneal macrophages preprogrammed with different doses of IGF2, the isolated macrophages were treated with lipopolysaccharide (LPS) and interleukin-4/13 (IL-4/13), respectively, simulating classical activation pathway and alternative activation pathway to stimulate macrophages. Real-time quantitative PCR (Real-time PCR, RT-PCR) results have shown that the expression of inflammatory factors in macrophages classically activated by lipopolysaccharide is inhibited by low-dose IGF2 (L-IGF2), and high-dose IGF2 has no significant regulation of some genes (Fig. 2D); the expression of anti-inflammatory factors in interleukin 4/13-activated macrophages is amplified by IGF2 (L-IGF2), and high-dose IGF2 (H-IGF2) has no significant effect (Fig. 2E).

These results suggest that the oxidative phosphorylation energy metabolism preference induced by low-dose IGF2 can promote a strong anti-inflammatory phenotype in macrophages, while high-dose IGF2 reverses the effect of low-dose IGF2, returning the energy metabolism pattern to aerobic glycolysis-dominated pattern close to that of the control group and reversing the inflammatory phenotype accordingly.

### Example 3 Low-dose IGF2 inhibits V-type ATPase activity, reduces lysosomal H⁺ concentration, promotes cytoplasmic H⁺ concentration, and increases mitochondrial inner membrane potential.

Oxidative phosphorylation occurs in the mitochondria, while aerobic glycolysis occurs in the cytoplasm. To explore how low doses of IGF2 enhance macrophage oxidative phosphorylation capacitation preference, we investigated cellular mitochondria. It is found that peritoneal macrophages (maturing and matured macrophages) derived from low-dose IGF2 (L-IGF2, 50 ng per mouse) treatment have both exhibited higher mitochondrial membrane potential (Fig. 3A), suggesting the significantly increased mitochondrial membrane potential has provided sufficient H⁺ substrate for oxidative phosphorylation of macrophages under the regulation of low-dose IGF2. Meanwhile, low-dose IGF2-treated peritoneal macrophages in mice with peritonitis has all displayed a lower cytoplasmic pH, even close to pH 5.5 (Fig. 3B). Under normal circumstances, the cytoplasmic H⁺ concentration is tightly regulated by V-type ATPases in the outer membrane and acidic organelles, and the pH of the cytoplasm is usually around 7.4. To verify whether the upregulation of cytoplasmic H⁺ concentration is caused by defective acidic organelle function, we examined the pH of the most important organelle responsible for the regulation of H⁺ balance, the lysosome. The study has found that the lysosomal pH of macrophages under the regulation of low-dose IGF2 is up-regulated to 6.5, which is significantly higher than that of the lysosomes of macrophages in the control and high-dose IGF2 groups (about 4.5-5.5) (Figure 3C). In addition, cell lysosomes were isolated and the activity of V-type ATPase on lysosomes was detected. It is found that low-dose IGF2 has significantly reduced the activity of V-type ATPase in macrophages, while high-dose IGF2 has no significant effect on V-type ATPase activity (Fig. 3D).

### Example 4 Construction of igf2r gene-deficient mice and igf1r or igf2r conditional knockout mice.

IGF2 can bind to IGF1R and IGF2R, and has the greatest affinity with IGF2R. When IGF2 binds to IGF2R, IGF2R is internalized, and IGF2 will be transported to lysosomes for degradation. Therefore, IGF2R is often considered to be the decoy receptor of IGF2, while IGF2 binds to IGF1R to function as a mitogen, promoting cell proliferation and survival². Based on the studies in the previous section, we have found that high-dose IGF2 has exhibited mitogen function to promote the proliferation and survival of peritoneal macrophages in mice with peritonitis, but low-dose IGF2 does not. Also in view of the maximum affinity of IGF2 to IGF2R, we have hypothesized that low-dose IGF2 preferentially binds to IGF2R and induces IGF2R-dependent biological effects, such as the occurrence of non-classical mitochondrial dynamics, and high-dose IGF2 still has the opportunity to bind to IGF1R after "depleting" the outer membrane IGF2R, counteracting the biological effect of IGF2R, and then promoting the proliferation and survival of peritoneal macrophages in peritonitis mice.

In order to study which receptors IGF2 depends on to realize the bidirectional regulation of monocytes, we constructed genetically engineered mice. In *igf2r* gene knockout mice, we used Crisper-Cas9 technology to target and knock out 2 bp or 13 bp bases in exon 6 of *igf2* gene, resulting in frameshift mutation to complete (Fig. 4A). Given that the complete deletion of the *igf2r* gene can cause mouse embryonic lethality, we finally obtained the *igf2r* gene knockout heterozygous mice-IGF2R^{+/-} mice for breeding conservation and experiments. The genotype identification of the offspring of IGF2R^{+/-}mice depends on Sequencing alignment (Figure 4B). We induced mouse peritoneal macrophages and used flow cytometry to detect the expression of IGF2R in these cells, confirming the knockout efficiency (Fig. 4C).

In order to construct *igf2r* conditional knockout mice, we used ES cell targeting technology to introduce loxp sequences in the same direction at both ends of the second exon of igf2r gene, and firstly constructed IGF2R^{*fl*/*fl*} mice. IGF1R^{*fl*/*fl*} mice and IGF2R^{*fl*/*fl*} mice were then mated with Lyz2*^{Cre}* mice, in which Lyz2 was expressed at high levels in myeloid cells¹⁸, until IGF1R^{*fl*/*fl*}Lyz2*^{Cre}* homozygous mice and IGF2R^{*fl*/*fl*}Lyz2*^{Cre}* homozygous mice were obtained (Fig. 4D, G). DNA level and protein level identification results confirmed the knockout efficiency (Fig. 4E, F, H, I).

### Example 5 Directed activation of IGF2R in monocytes inhibits inflammatory bowel disease.

To evaluate the role of IGF2-IGF2R-regulated monocytes and macrophages in other models of inflammation, we induced a model of inflammatory bowel disease using dextran sulfate sodium salt (DSS), an experimental animal model with the most similar symptoms of human Crohn's disease and ulcerative colitis^{19,20}. Next, we treated DSS-induced inflammatory bowel disease with different doses of IGF2, IGF2 mutants, and IGF2-treated peritoneal macrophages.

### 5.1 Low-dose IGF2 inhibits DSS-induced inflammatory bowel disease

We have found that low-dose IGF2 (L-IGF2, 50 ng per mouse) can significantly slow down DSS-induced weight loss in mice and significantly prolong mouse survival, however, high-dose IGF2 (H-IGF2, 1000 ng per mouse) has accelerated the weight loss of mice and can not prolong the survival time of mice (Fig. 5A, B). In addition, we also recorded and evaluated other indicators in mice, such as "Stool Score" and "Bleeding Score" and colon length, and have found that low-dose IGF2 can significantly reduce the "Stool Score" and "Bleeding Score" and better preserves the length of the mouse colon (Fig. 5C-E).

In histopathological analysis, we have found that the colonic villus of the control and high-dose IGF2-treated mice were largely destroyed, while the low-dose IGF2 group was relatively intact (Fig. 5F). In the results of TUNEL immunohistochemical staining, we have found that dead cells spread along the colonic villus to the basal layer, and the positive death signal in the control group and high-dose IGF2-treated group is significantly stronger than that in the low-dose IGF2-treated group (Fig. 5G). In the results of Ki67 immunohistochemical staining, we have found that the positive proliferation signal of Ki67 extends from the basement membrane to the villus, and the positive signal of Ki67 in the low-dose IGF2 group is significantly stronger than that in the control and high-dose IGF2-treated groups, indicating that monocytes and macrophages near the basement membrane play a role in the repair of intestinal villus, and macrophages at the colonic basement membrane of mice treated with low-dose IGF2 can better promote the repair of colonic villus (Figure 5H).

In conclusion, low-dose IGF2 can significantly improve the quality of life and survival time of DSS-induced inflammatory bowel disease mice, while high-dose IGF2 has no therapeutic effect and even aggravates some symptoms.

### 5.2 IGF2 treatment of inflammatory bowel disease is dependent on macrophages

To verify the critical role of IGF2-treated monocytes and macrophages in the treatment of inflammatory bowel disease, we utilized PBS, low-dose IGF2 (L-IGF2, 50 ng per mouse), and high-dose IGF2 (H-IGF2, 1000 ng per mouse) treated macrophages to treat DSS-induced inflammatory bowel disease in mice. The results show that both PBS and high-dose IGF2-treated peritoneal macrophages aggravates the weight loss of mice and shortens the survival time of mice when treating inflammatory bowel disease mice, while low-dose IGF2-treated peritoneal macrophages significantly improves the state of inflammatory bowel disease mice, significantly improves body mass index, and prolongs survival (Fig. 6A, B).

Therefore, IGF2-regulated macrophages at low doses can alleviate inflammatory bowel disease, while high-dose IGF2-regulated macrophages aggravate inflammatory bowel disease progression.

### 5.3 Low-dose IGF2 reduces pathogenic macrophage infiltration in the colon

Given the important role that macrophages play in DSS-induced inflammatory bowel disease, we performed numerical and phenotypic analyses of colon-infiltrating mononuclear cells. The results show that low-dose IGF2 (L-IGF2, 50 ng per mouse) treatment significantly reduces mononuclear cell infiltration in the colon, whereas high-dose IGF2 (H-IGF2, 1000 ng per mouse) increases mononuclear cell infiltration in colon tissue (Fig. 7A). This phenomenon is similar to the number of available peritoneal macrophages in low-dose and high-dose IGF2-treated peritonitis mice.

Before examining the phenotype of mononuclear cells isolated from colon tissue of mice with inflammatory bowel disease, we stimulated these cells with lipopolysaccharide for 5 hours and added Brefeldin A (BFA) to inhibit intracellular factor release. The experimental results show that macrophages in colon tissue of low-dose IGF2-treated mice with inflammatory bowel disease expresses less interleukin-1β (IL-1β) relative to cells in controls, whereas macrophages in the colon tissue of colitis mice treated with high-dose IGF2 significantly overexpresses IL-1β. (Fig. 7B, C). At the same time, we have analyzed the expression level of PD-L1 in macrophages, and found that the expression of PD-L1 on macrophages in the low-dose IGF2-treated group is significantly increased (Fig. 7D).

These results suggest that low-dose IGF2 inhibits IL-1β levels in colonic tissue-infiltrating macrophages in mice with inflammatory bowel disease, up-regulates the level of PD-L1, and decreases the number of macrophage infiltration, while high-dose IGF2 treatment aggravates this pathogenic phenotype of macrophages and increases the number of infiltration of this group of cells.

### 5.4 IGF1 aggravates inflammatory bowel disease

IGF2 preferentially binds to IGF2R at low doses and can bind to IGF1R at high doses. While IGF1 has the ability to bind IGF1R, but not IGF2R. In order to demonstrate the regulation of IGF1R activation on the inflammatory response, the study used IGF1 to treat inflammatory bowel disease. The study has found that both low-dose IGF1 (L-IGF1, 50 ng per mouse) and high-dose IGF1 (H-IGF2, 1000 ng per mouse) can significantly shorten the survival time of DSS-induced inflammatory bowel disease mice, significant shortening of colon length, and the colonic lamina propria macrophages expresses increased levels of IL-1β and decreased levels of PD-L1 (Figure 8A-D). In addition, the study also analyzed tthe production of nitric oxide and lactate and the consumption of glucose by peritoneal macrophages stimulated by LPS after low and high doses of IGF1 in peritonitis mice (Figure 8 E-G).The study has found that IGF1 significantly promotes nitric oxide production, lactate secretion, and glucose consumption in macrophages, suggesting that IGF1 performs pro-inflammatory functions through IGF1R.

### 5.5 IGF2R performs low-dose IGF2-induced anti-inflammatory macrophage function

In order to verify whether the regulation of low-dose IGF2 on the anti-inflammatory function of macrophages depends on IGF2R, the study used myeloid cell-specific knockout IGF2R mice to establish a peritonitis model, and analyzed the expression levels of PD-L1, IL-1β and cytoplasmic H⁺ in peritoneal macrophages with and without low-dose IGF2 (L-IGF2, 50 ng per mouse) injection. Studies have found that low-dose IGF2 can effectively promote the expression of PD-L1 on the surface of macrophages, inhibit the expression of IL-1β, and up-regulate the concentration of H⁺ in the cytoplasm of macrophages. When myeloid cells were depleted of IGF2R, the regulatory effects of low-dose IGF2 on macrophage expression of PD-L1, IL-1β, and cytoplasmic H⁺ concentrations are abolished (Figure 9A-C). Therefore, low-dose IGF2 modulates the anti-inflammatory properties of macrophages through IGF2R.

### 5.6 IGF2R performs the anti-inflammatory task of low-dose IGF2

In order to verify whether the mechanism of action of IGF2 in the treatment of DSS-induced inflammatory bowel disease in mice is consistent with our previous findings, we induced inflammatory bowel disease in mice with specific knockout of IGF2R or IGF1R in myeloid cells and treated with low-dose IGF2 (L-IGF2, 50 ng per mouse). The results have shown that IGF2R^{*fl*/*fl*}Lyz2*^{Cre}* mice with a specific knockout of IGF2R are more sensitive to inflammatory bowel disease and have more significant weight loss compared to control IGF2R^{*fl*/*fl*} mice, and low-dose IGF2 no longer plays an effective role against inflammatory bowel disease, the body weight continues to decrease, the survival time of mice is significantly shortened, the stool score and the degree of blood in the stool ar significantly aggravated, and the colonic inflammatory infiltration is significantly increased (Figure 10A-F).

Correspondingly, compared with the control group IGF1R^{*fl*/*fl*} mice, IGF1R^{*fl*/*fl*}Lyz2*^{Cre}* mice with specific knockout of IGF1R are more resistant to inflammatory bowel disease, whether in the PBS-treated group or high-dose IGF2 (H-IGF2, 1000 ng per mouse) treatment group, the body weight of the mice is significantly higher than that of the control group IGF1R^{*fl*/*fl*} mice under the corresponding treatment conditions, the survival of the mice is significantly prolonged, the stool score and the degree of blood in the stool are significantly reduced, and the colonic inflammatory infiltration is significantly reduced. (Fig. 10G-L).

The above experimental results show that in the DSS-induced inflammatory bowel disease mouse model, IGF2 treatment still relies on IGF2R to perform anti-inflammatory phenotype modeling, and IGF1R-activated signaling promotes the pro-inflammatory phenotype of macrophages in inflammatory bowel disease.

### 5.7 Leu27-IGF2 targeted activation of IGF2R can most effectively inhibit peritonitis and inflammatory bowel disease

In order to facilitate the study of the functions of IGF2R and IGF1R, many IGF2 mutants have been designed and reported, including IGF2 mutants that can selectively bind to IGF2R. The researchers mutated the tyrosine on position 27 of the IGF2 peptide to leucine to prepare a Leu27-IGF2 mutant (L27-IGF2). The affinity of the Leu27-IGF2 mutant to IGF2R and IGFBP proteins is unchanged, and the affinity for IGF1R is extremely significantly reduced.

Compared with wild-type IGF2, Leu27-IGF2 can significantly reduce the relative cytoplasmic pH of peritoneal monocytes and peritoneal macrophages in peritonitis mice, even at a high dose of 2000 ng/ml, we also compared the effect of Leu27-IGF2 on cytoplasmic pH at low doses, excluding the possibility of low Leu27-IGF2 titers (Fig. 11A). In a study of peritonitis, we have found that macrophages treated with both low-dose Leu27-IGF2 and high-dose Leu27-IGF2 have exhibited a significant decrease in nitric oxide (FIG. 11B) and lactate (FIG. 11C) production in response to LPS stimulation. At the same time, low- and high-dose Leu27-IGF2 treatment of macrophages show a significant inhibition of IL-1β levels and a marked increase in PD-L1 levels (Figure 11D-E). This indicates that both low-dose Leu27-IGF2 and high-dose Leu27-IGF2 can confer an anti-inflammatory phenotype on peritonitis macrophages in mice with peritonitis.

Similarly, in the DSS-induced inflammatory bowel disease mouse model, both low and high doses of Leu27-IGF2 can significantly inhibit body weight loss and improve mouse survival in inflammatory bowel disease mice (Figure 11F-G). The fecal scores, blood in the stool and colon length of these mice can also reflect the therapeutic effect of low and high doses of Leu27-IGF2 on inflammatory bowel disease (Figure 11H-J). Taking the above results together, we have found that the threshold for effective inhibition of inflammation by Leu27-IGF2 is increased by more than 20-fold relative to wild-type IGF2 (Fig. 11K).

In this part of the study, we validated the therapeutic effect of IGF2 in a mouse model of DSS-induced inflammatory bowel disease. It is confirmed that the effective inhibition of inflammatory bowel disease by low-dose IGF2 relies on the same mechanism of action as low-dose IGF2 in the treatment of peritonitis, and in both validated models, the IGF2R-dependent pathway can confer immune memory that significantly inhibits inflammation in macrophages. IGF2 mutants target the activation of IGF2R and exert the most stable anti-inflammatory effect.

### 5.8 Therapeutic effects of IGF2 mutants on inflammatory bowel disease

Human Leu27-IGF2 is an analog of IGF-2, tyrosine is replaced by leucine at position 27 of the human IGF2 sequence. Compared with IGF2, the binding ability of Leu27-IGF2 to IGF-binding proteins (IGFBPs) in serum is unchanged, but the binding ability of Leu27-IGF2 to IGF1 receptor (IGF1R) is reduced by 10-20 times. Thus the specificity of Leu27-IGF2 binding to the IGF2 receptor (IGF2R) is enhanced. The IGF2 peptide segment is composed of four domains (Domains), A, B, C, and D. The deletion of the D domain (amino acids 62-67) will significantly enhance its affinity with IGFBP2, IGFBP3, IGFBP4 and IGFBP6, the affinity with IGFBP1 and IGFBP5 remained unchanged.

Considering that the combination of IGF2 and IGFBP can prolong the half-life of IGF2 and enhance the stability of IGF2, we deleted the D domain on the basis of Leu27-IGF2, and expected to obtain a low affinity for IGF1R, high selectivity for IGF2R, high affinity for IGFBP, and a more stable IGF2 analog - Leu27-Des [62-67]-IGF2. The present study has found that both low-dose (1 ng per mouse) and high-dose (5 ng per mouse) Leu27-Des[62-67]-IGF2 can significantly inhibit DSS-induced inflammatory bowel disease-induced weight loss in mice, and significantly prolong the survival of mice with inflammatory bowel disease (Fig. 12A,B).

### 5.9 Activation of IGF2R promotes its nuclear transport and regulates cytoplasmic H⁺ concentration in macrophages

The study has found that IGF2 can reduce the distribution of IGF2R on the cell membrane surface of THP1 cells, showing a dose-dependent characteristic. From a low dose of 5 ng/ml to a high dose of 1000 ng ml, the reduction of IGF2R on the cell membrane surface reached the maximum, and the dose of IGF2 continued to increase, the cell membrane surface IGF2R did not continue to decrease (Fig. 13 A, B). Using western blotting, it was confirmed that after IGF2 treatment of THP1 cells, the distribution of IGF2R in the nucleus is significantly increased (Fig. 13C). Blocking nuclear transport of IGF2R with Ivermectin inhibits the distribution of IGF2R in the nucleus (Fig. 13D). At the same time, blocking nuclear transport of IGF2R suppresses the marked increase in macrophage cytoplasmic H⁺ concentration induced by low-dose IGF2 (Fig. 13E). In addition, it is found that Leu27-Des[62-67]-IGF2 can significantly down-regulate the expression of IGF2R on THP1 cells (Fig. 13F). Therefore, targeted activation of IGF2R can promote its nuclear transport and promote the formation of anti-inflammatory macrophages, thereby anti-inflammatory.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

### References:

1 Yu, H. & Rohan, T. Role of the insulin-like growth factor family in cancer development and progression. Journal of the National Cancer Institute 92, 1472-1489 (2000).
2 Enguita-German, M. & Fortes, P. Targeting the insulin-like growth factor pathway in hepatocellular carcinoma. World journal of hepatology 6, 716-737, doi:10.4254/wjh.v6.i10.716 (2014).
3 Killian, J. K. & Jirtle, R. L. Genomic structure of the human M6P/IGF2 receptor. Mammalian genome : official journal of the International Mammalian Genome Society 10, 74-77 (1999).
4 Gary-Bobo, M., Nirde, P., Jeanjean, A., Morere, A. & Garcia, M. Mannose 6-phosphate receptor targeting and its applications in human diseases. Current medicinal chemistry 14, 2945-2953 (2007).
5 Costello, M., Baxter, R. C. & Scott, C. D. Regulation of soluble insulin-like growth factor II/mannose 6-phosphate receptor in human serum: measurement by enzyme-linked immunosorbent assay. The Journal of clinical endocrinology and metabolism 84, 611-617, doi:10.1210/jcem.84.2.5488 (1999).
6 Sheet, A. d. f. US, Department of Health and Human Services. (2016).
7 Borgelt, L. M. Immunity and autoimmune diseases. Women's Health Across the Lifespan: A Pharmacotherapeutic Approach (2010).
8 Hanayama R, T. M., Miwa K, Shinohara A, Iwamatsu A, Nagata S. Identification of a factor that links apoptotic cells to phagocytes. Nature 417(6885): 182-7 (2002).
9 Miksa, M., Amin, D., Wu, R., Ravikumar, T. S. & Wang, P. Fractalkine-induced MFG-E8 leads to enhanced apoptotic cell clearance by macrophages. Molecular medicine 13, 553-560, doi:10.2119/2007-00019.Miksa (2007).
10 Miksa, M. et al. Maturation-induced down-regulation of MFG-E8 impairs apoptotic cell clearance and enhances endotoxin response. International journal of molecular medicine 22, 743-748 (2008).
11 Ferrero-Miliani, L., Nielsen, O. H., Andersen, P. S. & Girardin, S. E. Chronic inflammation: importance of NOD2 and NALP3 in interleukin-lbeta generation. Clinical and experimental immunology 147, 227-235, doi:10.1111/j.1365-2249.2006.03261.x (2007).
12 A.H., A. A. B. L. "Ch.2 Innate Immunity". In Saunders (Elsevier) (ed.). Basic Immunology. Functions and disorders of the immune system (3rd ed.) (2009).
13 Hocking, P. M., Robertson, G. W. & Gentle, M. J. Effects of anti-inflammatory steroid drugs on pain coping behaviours in a model of articular pain in the domestic fowl. Research in veterinary science 71, 161-166, doi:10.1053/rvsc.2001.0510 (2001).
14 Menkes, C. J. Effects of disease-modifying anti-rheumatic drugs, steroids and non-steroidal anti-inflammatory drugs on acute-phase proteins in rheumatoid arthritis. British journal of rheumatology 32 Suppl 3, 14-18 (1993).
15 Ohuchi, S. T. S. S. A review of mechanism of action of steroid and non-steroid anti-inflammatory drugs. In: Willoughby D.A., Giroud J.P. (eds) Inflammation: Mechanisms and Treatment. Inflammation: Mechanisms and Treatment 4 (1980).
16 Warden, S. J. Prophylactic use of NSAIDs by athletes: a risk/benefit assessment. The Physician and sportsmedicine 38, 132-138, doi:10.3810/psm.2010.04.1770 (2010).
17 Kumeji TAKEUCHI, N. I., Noboru HASEGAWA, Yoko YOSHIDA, Kenji YAMADA, Hiroshi KOGO, Yoshio AIZAWA. Inhibitory action of non-steroidal anti-inflammatory drugs on prostaglandin synthesis and release. Folia Pharmacologica Japonica 76, 525-531 (1980).
18 Clausen, B. E., Burkhardt, C., Reith, W., Renkawitz, R. & Forster, I. Conditional gene targeting in macrophages and granulocytes using LysMcre mice. Transgenic research (1999).
19 Zigmond, E. et al. Ly6C hi monocytes in the inflamed colon give rise to proinflammatory effector cells and migratory antigen-presenting cells. Immunity 37, 1076-1090, doi:10.1016/j.immuni.2012.08.026 (2012).
20 Chassaing, B., Aitken, J. D., Malleshappa, M. & Vijay-Kumar, M. Dextran sulfate sodium (DSS)-induced colitis in mice. Current protocols in immunology 104, Unit 15 25, doi:10.1002/0471142735.im1525s104 (2014).

## Claims

1. Use of a non-IGF1R-binding substance for the preparation of a composition or preparation for preventing and/or treating inflammatory diseases.

2. The use of claim 1, wherein the non-IGF1R-binding substance is selected from the group consisting of an IGF2 mutant, a vector expressing an IGF2 mutant, an antibody, a small molecule compound, and a combination thereof.

3. The use of claim 1, wherein the inflammatory disease is selected from the group consisting of: peritonitis, inflammatory bowel disease, multiple sclerosis, diabetes, systemic lupus erythematosus, scleroderma, hashimoto thyroiditis, autoimmune hepatitis, autoimmune uveitis, interstitial lung disease, psoriasis, leukoderma, dermatomyositis, kawasaki disease, adult ear disease, ankylosing spondylitis, sarcoidosis, enthesitis-related arthritis, Polyarticular Course Juvenile Idiopathic Arthritis, rheumatoid arthritis, graft-versus-host disease, autoimmune pancreatitis, Parkinson's disease, Alzheimer's disease, and a combination thereof.

4. The use of claim 1, wherein the non-IGF1R-binding substances include substances with higher selectivity or affinity for IGF2R than IGF1R, and substances that do not activate or substantially do not activate IGF1R that can efficiently activate IGF2R.

5. The use of claim 2, wherein the IGF2 mutant is mutated at the tyrosine corresponding to position 27 of SEQ ID NO.: 1 of the wild-type IGF2 protein.

6. The use of claim 2, wherein the IGF2 mutant is mutated to leucine at the tyrosine corresponding to position 27 of SEQ ID NO.: 1 of the wild-type IGF2 protein and optional deletion of the D-domain (threonine on position 62 to glutamic acid on position 67 of SEQ ID NO.: 1).

7. The use of claim 2, wherein the IGF2 mutant is selected from the group consisting of:
(a) a polypeptide having the amino acid sequence shown in any of SEQ ID NO.:2-58;
(b) a polypeptide derived from (a), which is formed by the substitution, deletion or addition of one or more (e.g., 2, 3, 4 or 5) amino acid residues in any of the amino acid sequences as shown in SEQ ID NO.: 2-58, and having anti-inflammatory activity.

8. A cell preparation, comprising:
a phagocyte treated with a non-IGF1R-binding substance.

9. The cell preparation of claim 8, wherein the phagocyte is selected from the group consisting of a monocyte, macrophage, monocyte precursor cell, and a combination thereof.

10. The cell preparation of claim 8, in the composition, the concentration of the phagocyte is 1×10⁴-5×10⁷/ml, preferably 5×10⁴-5×10⁶/ml, more preferably 1×10⁵-1×10⁶/ml.

11. A kit, comprising:
(i) a first container, and the active ingredient (a) a phagocyte treated with a non-IGF1R-binding substance, or a medicament containing the active ingredient (a) contained in the first container;
(ii) an optional second container, and the active ingredient (b) a non-IGF1R-binding substance, or a medicament containing the active ingredient (b), contained in the second container; and
(iii) instructions for preventing and/or treating inflammatory diseases by co-administering active ingredient (a) and active ingredient (b).

12. A method for screening a medicament, comprising the steps of:
(a) in a test group, adding a test substance to a cell culture system, and observing the binding activity of the test substance to IGF1R and/or IGF2R in the cell of the test group; in the control group, in a control group, no test substance is added to the culture system of the same cell;
wherein, if the binding activity of the test substance to IGF1R in the cell of the test group is decreased; and the binding activity to IGF2R is unchanged or increased, indicating that the test substance is a non-IGF1R-binding IGF2 mutant.

13. A method for screening a candidate for preventing and/or treating inflammatory diseases, comprising:
(a) in a test group, adding a test substance to a cell culture system, and observing the effect of the test substance on the expression and/or activity of IGF2R on the cell surface in the cell of the test group; and/or the effect of the test substance on nuclear transport of IGF2R; and/or the effect of the test substance on the redistribution of IGF2R to the cell nucleus; in a control group, the test substance is not added to the culture system of the same cell;
wherein if the test substance of the test group inhibits the expression and/or activity of IGF2R on the cell surface; and/or the test substance increases nuclear transport of IGF2R; and/or the test substance promotes the redistribution of IGF2R to the cell nucleus, indicating that the test substance is a candidate for preventing and/or treating inflammatory diseases.

14. A method for screening a medicament (or a method for screening a candidate), comprising:
(a) in a test group, adding a test substance to a cell culture system, and observing the effect of the test substance on the binding activity of IGF2 and IGF1R and/or IGF2R in the cell of the test group; in a control group, no test substance is added to the culture system of the same cell;
wherein if the test substance in the cell of the test group inhibits the binding activity of IGF2 and IGF1R; and having no effect on or promoting the binding activity of IGF2 and IGF2R, indicating that the test substance is a non-IGF1R-binding substance (or a candidate).

15. A method for obtaining a pro-inflammatory sample and an anti-inflammatory sample in vitro, comprising:
in a first set of experiments, treated with low doses of IGF2, thereby obtaining an anti-inflammatory sample; and
in a second set of experiments, treated with high doses of IGF2, thereby obtaining a pro-inflammatory sample.

16. A composition, comprising:
(a) the non-IGF1R-binding IGF2 mutant or candidate obtained according to the method of claim 12 or 13 or 14; wherein the non-IGF1R-binding IGF2 mutant does not include an IGF2 mutant selected from the group consisting of wild-type IGF2, amino acid fragment at positions 25-91 of wild-type IGF2, IGF2 mutant in which the glutamic acid at position 12 is mutated to Asp, Ala, Gln, His, Arg, or Lys, phenylalanine at position 26 is mutated to IGF2 mutant for Ser, IGF2 mutant with tyrosine mutation at position 27 to Leu, IGF2 mutant with valine at position 43 mutated to Leu, IGF2 mutant with D-domain deleted, and a combination thereof.

17. A method for inhibiting inflammatory activity, comprising the steps of:
In the presence of a non-IGF1R-binding substance (or a candidate with IGF2R selectivity), culturing a phagocyte, thereby inhibiting inflammatory activity.

18. Use of the cell preparation of claim 8, the kit of claim 11, or the composition of claim 16 for the preparation of a medicament for preventing and/or treating an inflammatory disease.


Sequence listing

<110>  SHANGHAI INSTITUTE OF NUTRITION AND HEALTH, CHINESE ACADEMY OF
SCIENCES

<120>  APPLICATION OF NON-IGF1R-BINDING SUBSTANCE IN PREVENTION AND/OR
TREATMENT OF INFLAMMATORY DISEASES

<130>  P2020-1194

<150>  CN201910854478.3
<151>  2019-09-10

<160>  80

<170>  PatentIn version 3.5

<210>  1
<211>  67
<212>  PRT
<213>  Homo sapiens

<400>  1

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  2
<211>  67
<212>  PRT
<213>  artificial sequence

<220>
<223>  the IGF2 mutant

<400>  2

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Leu Phe Ser Arg Pro Ala
            20                  25                  30

Ser Arg Val Ser Arg Arg Ser Arg Gly Ile Val Glu Glu Cys Cys Phe

35                  40                  45

Arg Ser Cys Asp Leu Ala Leu Leu Glu Thr Tyr Cys Ala Thr Pro Ala
    50                  55                  60

Lys Ser Glu
65

<210>  3
<211>  61
<212>  PRT
<213>  artificial sequence

<220>
<223>  the IGF2 mutant

<400>  3

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Leu Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  4
<211>  67
<212>  PRT
<213>  artificial sequence

<220>
<223>  the IGF2 mutant

<400>  4

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Asp Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210> 5
<211> 61
<212> PRT
<213> artificial sequence

<220>
<223> the IGF2 mutant

<400> 5

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Asp Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210> 6
<211> 67
<212> PRT
<213> artificial sequence

<220>
<223> the IGF2 mutant

<400> 6

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Ala Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210> 7
<211> 61
<212> PRT

<213>  artificial sequence

<220>
<223>  the IGF2 mutant

<400>  7

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Ala Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  8
<211>  67
<212>  PRT
<213>  artificial sequence

<220>
<223>  the IGF2 mutant

<400>  8

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Gln Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  9
<211>  61
<212>  PRT
<213>  artificial sequence

<220>
<223>  the IGF2 mutant

<400>  9

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Gln Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  10
<211>  67
<212>  PRT
<213>  artificial sequence

<220>
<223>  the IGF2 mutant

<400>  10

Ala Tyr Arg Pro Ser Glu Thr Leu Cys Gly Gly Glu Leu Val Asp Thr
1               5                   10                  15

Leu Gln Phe Val Cys Gly Asp Arg Gly Phe His Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  11
<211>  61
<212>  PRT
<213>  artificial sequence

<220>
<223>  the IGF2 mutant

<400>  11

Ala Tyr Arg Pro Ser Glu Thr Leu Cys Gly Gly Glu Leu Val Asp Thr
1               5                   10                  15

Leu Gln Phe Val Cys Gly Asp Arg Gly Phe His Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  12
<211>  67
<212>  PRT
<213>  artificial sequence

<220>
<223>  the IGF2 mutant

<400>  12

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Arg Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  13
<211>  61
<212>  PRT
<213>  artificial sequence

<220>
<223>  the IGF2 mutant

<400>  13

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Arg Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  14
<211>  67
<212>  PRT
<213>  artificial sequence

<220>
<223>  the IGF2 mutant

<400>  14

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Lys Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  15
<211>  61
<212>  PRT
<213>  artificial sequence

<220>
<223>  the IGF2 mutant

<400>  15

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Lys Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  16
<211>  67
<212>  PRT
<213>  artificial sequence

<220>
<223>  the IGF2 mutant

<400>  16

Ala Tyr Arg Pro Ser Glu Thr Leu Cys Gly Gly Asp Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  17
<211>  61
<212>  PRT
<213>  artificial sequence

<220>
<223>  the IGF2 mutant

<400>  17

Ala Tyr Arg Pro Ser Glu Thr Leu Cys Gly Gly Asp Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  18
<211>  67
<212>  PRT
<213>  artificial sequence

<220>
<223>  the IGF2 mutant

<400>  18

Ala Tyr Arg Pro Ser Glu Thr Leu Cys Gly Gly Ala Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210> 19
<211> 61
<212> PRT
<213> artificial sequence

<220>
<223> the IGF2 mutant

<400> 19

Ala Tyr Arg Pro Ser Glu Thr Leu Cys Gly Gly Ala Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210> 20
<211> 67
<212> PRT
<213> artificial sequence

<220>
<223> the IGF2 mutant

<400> 20

Ala Tyr Arg Pro Ser Glu Thr Leu Cys Gly Gly Gln Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Val Glu Glu Cys Cys Phe

35                  40                  45

Arg Ser Cys Asp Leu Ala Leu Leu Glu Thr Tyr Cys Ala Thr Pro Ala
    50                  55                  60

Lys Ser Glu
65

<210>  21
<211>  61
<212>  PRT
<213>  artificial sequence

<220>
<223>  the IGF2 mutant

<400>  21

Ala Tyr Arg Pro Ser Glu Thr Leu Cys Gly Gly Gln Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  22
<211>  67
<212>  PRT
<213>  artificial sequence

<220>
<223>  the IGF2 mutant

<400>  22

Ala Tyr Arg Pro Ser Glu Thr Leu Cys Gly Gly His Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65


<210> 23
<211> 61
<212> PRT
<213> artificial sequence

<220>
<223> the IGF2 mutant

<400> 23

Ala Tyr Arg Pro Ser Glu Thr Leu Cys Gly Gly His Leu Val Asp Thr
1               5                   10                  15


Leu Gln Phe Val Cys Gly Asp Arg Gly Phe Tyr Phe Ser Arg Pro Ala
            20                  25                  30


Ser Arg Val Ser Arg Arg Ser Arg Gly Ile Val Glu Glu Cys Cys Phe
        35                  40                  45


Arg Ser Cys Asp Leu Ala Leu Leu Glu Thr Tyr Cys Ala
    50                  55                  60


<210> 24
<211> 67
<212> PRT
<213> artificial sequence

<220>
<223> the IGF2 mutant

<400> 24

Ala Tyr Arg Pro Ser Glu Thr Leu Cys Gly Gly Arg Leu Val Asp Thr
1               5                   10                  15


Leu Gln Phe Val Cys Gly Asp Arg Gly Phe Tyr Phe Ser Arg Pro Ala
            20                  25                  30


Ser Arg Val Ser Arg Arg Ser Arg Gly Ile Val Glu Glu Cys Cys Phe
        35                  40                  45


Arg Ser Cys Asp Leu Ala Leu Leu Glu Thr Tyr Cys Ala Thr Pro Ala
    50                  55                  60


Lys Ser Glu
65


<210> 25
<211> 61
<212> PRT

<213>  artificial sequence

<220>
<223>  the IGF2 mutant

<400>  25

Ala Tyr Arg Pro Ser Glu Thr Leu Cys Gly Gly Arg Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  26
<211>  67
<212>  PRT
<213>  artificial sequence

<220>
<223>  the IGF2 mutant

<400>  26

Ala Tyr Arg Pro Ser Glu Thr Leu Cys Gly Gly Lys Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  27
<211>  61
<212>  PRT
<213>  artificial sequence

<220>
<223>  the IGF2 mutant

<400>  27

Ala Tyr Arg Pro Ser Glu Thr Leu Cys Gly Gly Lys Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  28
<211>  67
<212>  PRT
<213>  artificial sequence

<220>
<223>  the IGF2 mutant

<400>  28

Ala Tyr Arg Pro Ser Glu Thr Leu Cys Gly Gly Glu Leu Val Asp Thr
1               5                   10                  15

Leu Gln Phe Val Cys Gly Asp Arg Gly Ser Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  29
<211>  61
<212>  PRT
<213>  artificial sequence

<220>
<223>  the IGF2 mutant

<400>  29

Ala Tyr Arg Pro Ser Glu Thr Leu Cys Gly Gly Glu Leu Val Asp Thr
1               5                   10                  15

Leu Gln Phe Val Cys Gly Asp Arg Gly Ser Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  30
<211>  67
<212>  PRT
<213>  artificial sequence

<220>
<223>  the IGF2 mutant

<400>  30

Ala Tyr Arg Pro Ser Glu Thr Leu Cys Gly Gly Glu Leu Val Asp Thr
1               5                   10                  15

Leu Gln Phe Val Cys Gly Asp Arg Gly Asp Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  31
<211>  61
<212>  PRT
<213>  artificial sequence

<220>
<223>  the IGF2 mutant

<400>  31

Ala Tyr Arg Pro Ser Glu Thr Leu Cys Gly Gly Glu Leu Val Asp Thr
1               5                   10                  15

Leu Gln Phe Val Cys Gly Asp Arg Gly Asp Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  32
<211>  67
<212>  PRT
<213>  artificial sequence

<220>
<223>  the IGF2 mutant

<400>  32

Ala Tyr Arg Pro Ser Glu Thr Leu Cys Gly Gly Glu Leu Val Asp Thr
1               5                   10                  15

Leu Gln Phe Val Cys Gly Asp Arg Gly Ala Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  33
<211>  61
<212>  PRT
<213>  artificial sequence

<220>
<223>  the IGF2 mutant

<400>  33

Ala Tyr Arg Pro Ser Glu Thr Leu Cys Gly Gly Glu Leu Val Asp Thr
1               5                   10                  15

Leu Gln Phe Val Cys Gly Asp Arg Gly Ala Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  34
<211>  67
<212>  PRT
<213>  artificial sequence

<220>
<223>  the IGF2 mutant

<400>  34

Ala Tyr Arg Pro Ser Glu Thr Leu Cys Gly Gly Glu Leu Val Asp Thr
1               5                   10                  15

Leu Gln Phe Val Cys Gly Asp Arg Gly Gln Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  35
<211>  61
<212>  PRT
<213>  artificial sequence

<220>
<223>  the IGF2 mutant

<400>  35

Ala Tyr Arg Pro Ser Glu Thr Leu Cys Gly Gly Glu Leu Val Asp Thr
1               5                   10                  15

Leu Gln Phe Val Cys Gly Asp Arg Gly Gln Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  36
<211>  67
<212>  PRT
<213>  artificial sequence

<220>
<223>  the IGF2 mutant

<400>  36

Ala Tyr Arg Pro Ser Glu Thr Leu Cys Gly Gly Glu Leu Val Asp Thr
1               5                   10                  15

Leu Gln Phe Val Cys Gly Asp Arg Gly His Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  37
<211>  61
<212>  PRT
<213>  artificial sequence

<220>
<223>  the IGF2 mutant

<400>  37

Ala Tyr Arg Pro Ser Glu Thr Leu Cys Gly Gly Glu Leu Val Asp Thr
1               5                   10                  15

Leu Gln Phe Val Cys Gly Asp Arg Gly His Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  38
<211>  67
<212>  PRT
<213>  artificial sequence

<220>
<223>  the IGF2 mutant

<400>  38

Ala Tyr Arg Pro Ser Glu Thr Leu Cys Gly Gly Glu Leu Val Asp Thr
1               5                   10                  15

Leu Gln Phe Val Cys Gly Asp Arg Gly Arg Tyr Phe Ser Arg Pro Ala
            20                  25                  30

Ser Arg Val Ser Arg Arg Ser Arg Gly Ile Val Glu Glu Cys Cys Phe

35                  40                  45

Arg Ser Cys Asp Leu Ala Leu Leu Glu Thr Tyr Cys Ala Thr Pro Ala
    50                  55                  60

Lys Ser Glu
65

<210>  39
<211>  61
<212>  PRT
<213>  artificial sequence

<220>
<223>  the IGF2 mutant

<400>  39

Ala Tyr Arg Pro Ser Glu Thr Leu Cys Gly Gly Glu Leu Val Asp Thr
1               5                   10                  15

Leu Gln Phe Val Cys Gly Asp Arg Gly Arg Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala
    50                  55                  60

<210>  40
<211>  67
<212>  PRT
<213>  artificial sequence

<220>
<223>  the IGF2 mutant

<400>  40

Ala Tyr Arg Pro Ser Glu Thr Leu Cys Gly Gly Glu Leu Val Asp Thr
1               5                   10                  15

Leu Gln Phe Val Cys Gly Asp Arg Gly Lys Tyr Phe Ser Arg Pro Ala
            20                  25                  30

Ser Arg Val Ser Arg Arg Ser Arg Gly Ile Val Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65


<210>  41
<211>  61
<212>  PRT
<213>  artificial sequence

<220>
<223>  the IGF2 mutant

<400>  41

Ala Tyr Arg Pro Ser Glu Thr Leu Cys Gly Gly Glu Leu Val Asp Thr
1               5                   10                  15


Leu Gln Phe Val Cys Gly Asp Arg Gly Lys Tyr Phe Ser Arg Pro Ala
            20                  25                  30


Ser Arg Val Ser Arg Arg Ser Arg Gly Ile Val Glu Glu Cys Cys Phe
        35                  40                  45


Arg Ser Cys Asp Leu Ala Leu Leu Glu Thr Tyr Cys Ala
    50                  55                  60


<210>  42
<211>  67
<212>  PRT
<213>  artificial sequence

<220>
<223>  the IGF2 mutant

<400>  42

Ala Tyr Arg Pro Ser Glu Thr Leu Cys Gly Gly Glu Leu Val Asp Thr
1               5                   10                  15


Leu Gln Phe Val Cys Gly Asp Arg Gly Ser Tyr Phe Ser Arg Pro Ala
            20                  25                  30


Ser Arg Val Ser Arg Arg Ser Arg Gly Ile Val Glu Glu Cys Cys Phe
        35                  40                  45


Arg Ser Cys Asp Leu Ala Leu Leu Glu Thr Tyr Cys Ala Thr Pro Ala
    50                  55                  60


Lys Ser Glu
65


<210>  43
<211>  61
<212>  PRT

<213>  artificial sequence

<220>
<223>  the IGF2 mutant

<400>  43

Ala Tyr Arg Pro Ser Glu Thr Leu Cys Gly Gly Glu Leu Val Asp Thr
1               5                   10                  15

Leu Gln Phe Val Cys Gly Asp Arg Gly Ser Tyr Phe Ser Arg Pro Ala
            20                  25                  30

Ser Arg Val Ser Arg Arg Ser Arg Gly Ile Leu Glu Glu Cys Cys Phe
        35                  40                  45

Arg Ser Cys Asp Leu Ala Leu Leu Glu Thr Tyr Cys Ala
    50                  55                  60

<210>  44
<211>  61
<212>  PRT
<213>  artificial sequence

<220>
<223>  the IGF2 mutant

<400>  44

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Leu Glu Glu Cys Cys Phe
        35                  40                  45

Arg Ser Cys Asp Leu Ala Leu Leu Glu Thr Tyr Cys Ala
    50                  55                  60

<210>  45
<211>  67
<212>  PRT
<213>  artificial sequence

<220>
<223>  the IGF2 mutant

<400>  45

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Asp Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  46
<211>  61
<212>  PRT
<213>  artificial sequence

<220>
<223>  the IGF2 mutant

<400>  46

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Asp Glu Glu Cys Cys Phe
        35                  40                  45

Arg Ser Cys Asp Leu Ala Leu Leu Glu Thr Tyr Cys Ala
    50                  55                  60

<210>  47
<211>  67
<212>  PRT
<213>  artificial sequence

<220>
<223>  the IGF2 mutant

<400>  47

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Ala Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  48
<211>  61
<212>  PRT
<213>  artificial sequence

<220>
<223>  the IGF2 mutant

<400>  48

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Ala Glu Glu Cys Cys Phe
        35                  40                  45

Arg Ser Cys Asp Leu Ala Leu Leu Glu Thr Tyr Cys Ala
    50                  55                  60

<210>  49
<211>  67
<212>  PRT
<213>  artificial sequence

<220>
<223>  the IGF2 mutant

<400>  49

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Gln Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  50
<211>  61
<212>  PRT
<213>  artificial sequence

<220>
<223>  the IGF2 mutant

<400>  50

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Gln Glu Glu Cys Cys Phe
        35                  40                  45

Arg Ser Cys Asp Leu Ala Leu Leu Glu Thr Tyr Cys Ala
    50                  55                  60

<210>  51
<211>  67
<212>  PRT
<213>  artificial sequence

<220>
<223>  the IGF2 mutant

<400>  51

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile His Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  52
<211>  61
<212>  PRT
<213>  artificial sequence

<220>
<223>  the IGF2 mutant

<400>  52

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile His Glu Glu Cys Cys Phe
        35                  40                  45

Arg Ser Cys Asp Leu Ala Leu Leu Glu Thr Tyr Cys Ala
    50                  55                  60

<210>  53
<211>  67
<212>  PRT
<213>  artificial sequence

<220>
<223>  the IGF2 mutant

<400>  53

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Arg Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  54
<211>  61
<212>  PRT
<213>  artificial sequence

<220>
<223>  the IGF2 mutant

<400>  54

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Arg Glu Glu Cys Cys Phe
        35                  40                  45

Arg Ser Cys Asp Leu Ala Leu Leu Glu Thr Tyr Cys Ala
    50                  55                  60

<210>  55
<211>  67
<212>  PRT
<213>  artificial sequence

<220>
<223>  the IGF2 mutant

<400>  55

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Lys Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210>  56
<211>  61
<212>  PRT
<213>  artificial sequence

<220>
<223>  the IGF2 mutant

<400>  56

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Tyr Phe Ser Arg Pro Ala
            20                  25                  30

Ser Arg Val Ser Arg Arg Ser Arg Gly Ile Lys Glu Glu Cys Cys Phe

        35                  40                  45

Arg Ser Cys Asp Leu Ala Leu Leu Glu Thr Tyr Cys Ala
    50                  55                  60

<210> 57
<211> 67
<212> PRT
<213> artificial sequence

<220>
<223> the IGF2 mutant

<400> 57

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Leu Phe Ser Arg Pro Ala
            20                  25                  30

Ser Arg Val Ser Arg Arg Ser Arg Gly Ile Leu Glu Glu Cys Cys Phe
        35                  40                  45

Arg Ser Cys Asp Leu Ala Leu Leu Glu Thr Tyr Cys Ala Thr Pro Ala
    50                  55                  60

Lys Ser Glu
65

<210> 58
<211> 61
<212> PRT
<213> artificial sequence

<220>
<223> the IGF2 mutant

<400> 58

Ala Tyr Arg Pro Ser Glu Thr Leu Cys Gly Gly Glu Leu Val Asp Thr
1               5                   10                  15

Leu Gln Phe Val Cys Gly Asp Arg Gly Phe Leu Phe Ser Arg Pro Ala
            20                  25                  30

Ser Arg Val Ser Arg Arg Ser Arg Gly Ile Leu Glu Glu Cys Cys Phe
        35                  40                  45

Arg Ser Cys Asp Leu Ala Leu Leu Glu Thr Tyr Cys Ala
    50                  55                  60

<210>  59
<211>  31
<212>  DNA
<213>  artificial sequence

<220>
<223>  primer

<400>  59
cgtgaagtct gtctatggaa gagactggac c                                    31


<210>  60
<211>  35
<212>  DNA
<213>  artificial sequence

<220>
<223>  primer

<400>  60
cattaccaag ctcacactcc cttctcttct ctatt                                35


<210>  61
<211>  24
<212>  DNA
<213>  artificial sequence

<220>
<223>  primer

<400>  61
ccccggtgtt gaggttgata gata                                            24


<210>  62
<211>  24
<212>  DNA
<213>  artificial sequence

<220>
<223>  primer

<400>  62
caatttaggg ctgaagcgat ggat                                            24


<210>  63
<211>  22
<212>  DNA
<213>  artificial sequence

<220>
<223>  primer

<400>  63
cttcccagct tgctactcta gg                                              22


<210>  64
<211>  22
<212>  DNA

<213>  artificial sequence

<220>
<223>  primer

<400>  64
caggcttgca atgagacatg gg                                                22


<210>  65
<211>  23
<212>  DNA
<213>  artificial sequence

<220>
<223>  primer

<400>  65
tgagacgtag cgagattgct gta                                               23


<210>  66
<211>  20
<212>  DNA
<213>  artificial sequence

<220>
<223>  primer

<400>  66
cccagaaatg ccagattacg                                                   20


<210>  67
<211>  21
<212>  DNA
<213>  artificial sequence

<220>
<223>  primer

<400>  67
cttgggctgc cagaatttct c                                                 21


<210>  68
<211>  20
<212>  DNA
<213>  artificial sequence

<220>
<223>  primer

<400>  68
ttacagtcgg ccaggctgac                                                   20


<210>  69
<211>  19
<212>  DNA
<213>  artificial sequence

<220>

<223>  primer

<400>  69
cctgtagccc acgtcgtag                                                    19


<210>  70
<211>  22
<212>  DNA
<213>  artificial sequence

<220>
<223>  primer

<400>  70
gggagtagac aaggtacaac cc                                                22


<210>  71
<211>  22
<212>  DNA
<213>  artificial sequence

<220>
<223>  primer

<400>  71
gcaactgttc ctgaactcaa ct                                                22


<210>  72
<211>  21
<212>  DNA
<213>  artificial sequence

<220>
<223>  primer

<400>  72
atcttttggg gtccgtcaac t                                                 21


<210>  73
<211>  21
<212>  DNA
<213>  artificial sequence

<220>
<223>  primer

<400>  73
ccaagtgctg ccgtcatttt c                                                 21


<210>  74
<211>  21
<212>  DNA
<213>  artificial sequence

<220>
<223>  primer

<400>  74

ggctcgcagg gatgatttca a                                                21

<210>  75
<211>  23
<212>  DNA
<213>  artificial sequence

<220>
<223>  primer

<400>  75
ccaatccagc taactatccc tcc                                              23

<210>  76
<211>  20
<212>  DNA
<213>  artificial sequence

<220>
<223>  primer

<400>  76
acccagtagc agtcatccca                                                  20

<210>  77
<211>  21
<212>  DNA
<213>  artificial sequence

<220>
<223>  primer

<400>  77
aggtccctat ggtgccaatg t                                                21

<210>  78
<211>  20
<212>  DNA
<213>  artificial sequence

<220>
<223>  primer

<400>  78
cggcaggatt ttgaggtcca                                                  20

<210>  79
<211>  22
<212>  DNA
<213>  artificial sequence

<220>
<223>  primer

<400>  79
gacttcagca ctcaagacat cc                                               22

<210>  80
<211>  21
<212>  DNA
<213>  artificial sequence

<220>
<223>  primer

<400>  80
ttgtgtcgcc aaaatgctag g                                                21